# EUROPÄISCHE PATENTSCHRIFT

(11) **EP 1 497 439 B1**
(45) Veröffentlichungstag und Bekanntmachung des Hinweises auf die Patenterteilung: **06.09.2006**
(21) Anmeldenummer: 03722417.7
(22) Anmeldetag: 08.04.2003
(51) Int. Cl.: C12N 15/861

(54) **REKOMBINANTE VIRALE VEKTOREN ZUR TETRACYCLINREGULIERBAREN GENEXPRESSION**
RECOMBINING VIRAL VECTORS FOR THE TETRACYCLINE-REGULATED EXPRESSION OF GENES
VECTEURS VIRAUX RECOMBINANTS POUR L'EXPRESSION GENIQUE REGULABLE PAR LA TETRACYCLINE

(30) Priorität: 15.04.2002 DE 10216800; 17.05.2002 DE 10222513
(43) Veröffentlichungstag der Anmeldung: 19.01.2005
(73) Patentinhaber: Universitätsklinikum Hamburg-Eppendorf, 20246 Hamburg (DE)
(72) Erfinder: BLOCK, Andreas, 22529 Hamburg (DE)
(74) Vertreter: Weber-Quitzau, Martin
(86) Internationale Anmeldenummer: PCT/EP2003/003638
(87) Internationale Veröffentlichungsnummer: WO 2003/087294

(56) Entgegenhaltungen:
- EP-A- 1 083 227
- EP-A- 1 092 771
- NAKAGAWA SHINSAKU ET AL: "Tetracycline-regulatable adenovirus vectors: Pharmacologic properties and clinical potential" EUROPEAN JOURNAL OF PHARMACEUTICAL SCIENCES, Bd. 13, Nr. 1, April 2001 (2001-04), Seiten 53-60, XP002258697 ISSN: 0928-0987
- STRATHDEE C A ET AL: "Efficient control of tetracycline-responsive gene expression from an autoregulated bi-directional expression vector" GENE, Bd. 229, Nr. 1-2, 18. März 1999 (1999-03-18), Seiten 21-29, XP004161154 ISSN: 0378-1119
- UNSINGER J ET AL: "Retroviral vectors for the transduction of autoregulated, bidirectional expression cassettes." MOLECULAR THERAPY: THE JOURNAL OF THE AMERICAN SOCIETY OF GENE THERAPY, Bd. 4, Nr. 5, November 2001 (2001-11), Seiten 484-489, XP002258698 ISSN: 1525-0016
- BARON U ET AL: "CO-REGULATION OF TWO GENE ACTIVITIES BY TETRACYCLINE VIA A BIDIRECTIONAL PROMOTER" NUCLEIC ACIDS RESEARCH, Bd. 23, Nr. 17, 11. September 1995 (1995-09-11), Seiten 3605-3606, XP000775822 ISSN: 0305-1048

### SEQUENZPROTOKOLL

<110> Universitätsklinikum Hamburg-Eppendorf
<120> Rekombinante virale Vektoren zur Tetracyclin-regulierbaren Genexpression
<130> P 63006
<160> 5
<170> PatentIn version 3.1
<210> 1
   <211> 11569
   <212> DNA
   <213> Artificial Sequence
<220>
   <223> Adenovirales Expressionsplasmid pAd.3r.hscIL-12 zur regulierten Expression des humanen IL-12
<220>
   <221> gene
   <222> (327)..(713)
   <223> VP16
<220>
   <221> gene
   <222> (714)..(1352)
   <223> TetR
<220>
   <221> Intron
   <222> (1353)..(1912)
   <223>
<220>
   <221> promoter
   <222> (1864)..(1902)
   <223> TK-min
<220>
   <221> protein_bind
   <222> (1913).. (2212)
   <223> TetO7
<220>
   <221> misc_feature
   <222> (2213)..(2709)
   <223> CMV-min + Intron
<220>
   <221> promoter
   <222> (2226)..(2264)
   <223> CMV-min
<220>
   <221> gene
   <222> (2710)..(4308)
   <223> Humanes single-chain IL-12
<220>
   <221> mutation
   <222> (4020)..(4023)
   <223> T/C, A/T, G/C, T/G
<400> 1
   ctgctggttc tttccgcctc agaagccata gagcccaccg catccccagc atgcctgcta     60
   ttgtcttccc aatcctcccc cttgctgtcc tgccccaccc caccccccag aatagaatga    120
   cacctactca gacaatgcga tgcaatttcc tcattttatt aggaaaggac agtgggagtg    180
   gcaccttcca gggtcaagga aggcacgggg gaggggcaaa caacagatgg ctggcaacta    240
   gaaggcacag tcgaggctga tcagcgagct ctagcattta ggtgacacta tagaataggg    300
   ccctctagga tcgatcctcg cgccccctac ccaccgtact cgtcaattcc aagggcatcg    360
   gtaaacatct gctcaaactc gaagtcggcc atatccagag cgccgtaggg ggcggagtcg    420
   tggggggtaa atcccggacc cggggaatcc ccgtccccca acatgtccag atcgaaatcg    480
   tctagcgcgt cggcatgcgc catcgccacg tcctcgccgt ctaagtggag ctcgtccccc    540
   aggctgacat cggtcggggg ggccgtggac agtctgcgcg tgtgtcccgc ggggagaaag    600
   gacaggcgcg gagccgccag ccccgcctct tcgggggcgt cgtcgtccgg gagatcgagc    660
   aggccctcga tggtagaccc gtaattgttt ttcgtacgcg cgcggctgta cgcggaccca    720
   ctttcacatt taagttgttt ttctaatccg catatgatca attcaaggcc gaataagaag    780
   gctggctctg caccttggtg atcaaataat tcgatagctt gtcgtaataa tggcggcata    840
   ctatcagtag taggtgtttc cctttcttct ttagcgactt gatgctcttg atcttccaat    900
   acgcaaccta aagtaaaatg ccccacagcg ctgagtgcat ataatgcatt ctctagtgaa    960
   aaaccttgtt ggcataaaaa ggctaattga ttttcgagag tttcatactg tttttctgta   1020
   ggccgtgtac ctaaatgtac ttttgctcca tcgcgatgac ttagtaaagc acatctaaaa   1080
   cttttagcgt tattacgtaa aaaatcttgc cagctttccc cttctaaagg gcaaaagtga   1140
   gtatggtgcc tatctaacat ctcaatggct aaggcgtcga gcaaagcccg cttatttttt   1200
   acatgccaat acaatgtagg ctgctctaca cctagcttct gggcgagttt acgggttgtt   1260
   aaaccttcga ttccgacctc attaagcagc tctaatgcgc tgttaatcac tttactttta   1320
   tctaatctag agggtctggg tctctttggc atggtcgaat taattcgcgt cgagccggcc   1380
   gcgggtacaa ttccggttgg acctgggagt ggacacctgt ggagagaaag gcaaagtgga   1440
   tgtcattgtc actcaagtgt atggccagat ctcaagcctg ccacacctca agcttgacaa   1500
   caaaaagatt gtcttttctg accagatgga cgcggccacc ctcaaaggca tcaccgcggg   1560
   ccaggtgaat atcaaatcct cctcgttttt ggaaactgac aatcttagcg cagaagtcat   1620
   gcccgctttt gagagggagt actcacccca acagtcgaga ggttttccga tccggtcgat   1680
   gcggactcgc tcaggtccct cggtggcgga gtaccgttcg gaggccgacg ggtttccgat   1740
   ccaagagtac tggaaagacc gcgaagagtt tgtcctcaac cgcgagccca acaggcgtcg   1800
   aagcttgatg ggtcgctcgg tgttcgaggc cacacgcgtc accttaatat gcgaagtgga   1860
   cctcggaccg cgccgccccg actgcatctg cgtgttcgaa ttgcccggcg agctcgactt   1920
   tcacttttct ctatcactga tagggagtgg taaactcgac tttcactttt ctctatcact   1980
   gatagggagt ggtaaactcg actttcactt ttctctatca ctgataggga gtggtaaact   2040
   cgactttcac ttttctctat cactgatagg gagtggtaaa ctcgactttc acttttctct   2100
   atcactgata gggagtggta aactcgactt tcacttttct ctatcactga tagggagtgg   2160
   taaactcgac tttcactttt ctctatcact gatagggagt ggtaaactcg acggtcgagg   2220
   gtcgagtagg cgtgtacggt gggaggccta tataagcaga gctcgtttag tgaaccgtca   2280
   gatcgcctgg agacgccatc cacgctgttt tgacctccat agaagacacc gggaccgatc   2340
   cagcctccgc ggccccgaat tgcgaagctt tattgcggta gtttatcaca gttaaattgc   2400
   taacgcagtc agtgcttctg acacaacagt ctcgaactta agctgcagaa gttggtcgtg   2460
   aggcactggg caggtaagta tcaaggttac aagacaggtt taaggagacc aatagaaact   2520
   gggcttgtcg agacagagaa gactcttgcg tttctgatag gcacctattg gtcttactga   2580
   catccacttt gcctttctct ccacaggtgt ccactcccag ttcaattaca gctcttaagg   2640
   ctagagtact taatacgact cactataggc tagcctcgag aattcacgcg tggtaccgag   2700
   ctcggatcca tgggtcacca gcagttggtc atctcttggt tttccctggt ttttctggca   2760
   tctcccctcg tggccatatg ggaactgaag aaagatgttt atgtcgtaga attggattgg   2820
   tatccggatg cccctggaga aatggtggtc ctcacctgtg acacccctga agaagatggt   2880
   atcacctgga ccttggacca gagcagtgag gtcttaggct ctggcaaaac cctgaccatc   2940
   caagtcaaag agtttggaga tgctggccag tacacctgtc acaaaggagg cgaggttcta   3000
   agccattcgc tcctgctgct tcacaaaaag gaagatggaa tttggtccac tgatatttta 3060
   aaggaccaga aagaacccaa aaataagacc tttctaagat gcgaggccaa gaattattct 3120
   ggacgtttca cctgctggtg gctgacgaca atcagtactg atttgacatt cagtgtcaaa 3180
   agcagcagag gctcttctga cccccaaggg gtgacgtgcg gagctgctac actctctgca 3240
   gagagagtca gaggggacaa caaggagtat gagtactcag tggagtgcca ggaggacagt 3300
   gcctgcccag ctgctgagga gagtctgccc attgaggtca tggtggatgc cgttcacaag 3360
   ctcaagtatg aaaactacac cagcagcttc ttcatcaggg acatcatcaa acctgaccca 3420
   cccaagaact tgcagctgaa gccattaaag aattctcggc aggtggaggt cagctgggag 3480
   taccctgaca cctggagtac tccacattcc tacttctccc tgacattctg cgttcaggtc 3540
   cagggcaaga gcaagagaga aaagaaagat agagtcttca cggacaagac ctcagccacg 3600
   gtcatctgcc gcaaaaatgc cagcattagc gtgcgggccc aggaccgcta ctatagctca 3660
   tcttggagcg aatgggcatc tgtgccctgc agtggtggcg gtggcggcgg atctagaaac 3720
   ctccccgtgg ccactccaga cccaggaatg ttcccatgcc ttcaccactc ccaaaacctg 3780
   ctgagggccg tcagcaacat gctccagaag gccagacaaa ctctagaatt ttacccttgc 3840
   acttctgaag agattgatca tgaagatatc acaaaagata aaaccagcac agtggaggcc 3900
   tgtttaccat tggaattaac caagaatgag agttgcctaa attccagaga gacctctttc 3960
   ataactaatg ggagttgcct ggcctccaga aagacctctt ttatgatggc cctgtgcctc 4020
   tcgagtattt atgaagactc gaagatgtac caggtggagt tcaagaccat gaatgcaaag 4080
   cttctgatgg atcctaagag gcagatcttt ctagatcaaa acatgctggc agttattgat 4140
   gagctgatgc aggccctgaa tttcaacagt gagactgtgc cacaaaaatc ctcccttgaa 4200
   gaaccggatt tttataaaac taaaatcaag ctctgcatac ttcttcatgc tttcagaatt 4260
   cgggcagtga ctattgatag agtgatgagc tatctgaatg cttcctaaaa agcgaggtcg 4320
   atccggatta gtccaatttg ttaaagacag gatatcagtg gtccaggctc tagttttgac 4380
   tcaacaatat caccagctga agcctataga gtacgagcca tagataaaat aaaagatttt 4440
   atttagtctc cagaaaaagg ggggaatgaa agaccccacc tgtaggtttg gcaagctagt 4500
   aacggccgcc agtgtgctgg aattctgcag atatccatca cactggcggc cgctcgagca 4560
   tgcatctaga gggccctatt ctatagtgtc acctaaatgc tagagctcgc tgatcagcct 4620
   cgactgtgcc ttctagttgc cagccatctg ttgtttgccc ctcccccgtg ccttccttga 4680
   ccctggaagg tgccactccc actgtccttt cctaataaaa tgaggaaatt gcatcgcatt 4740
   gtctgagtag gtgtcattct attctggggg gtggggtggg gcaggacagc aagggggagg 4800
   attgggaaga caatagcagg catgctgggg atgcggtggg ctctatggct tctgaggcgg 4860
   aaagaaccag tcgacatcga tgctagagtg gaaggtgctg aggtacgatg agacccgcac 4920
   caggtgcaga ccctgcgagt gtggcggtaa acatattagg aaccagcctg tgatgctgga 4980
   tgtgaccgag gagctgaggc ccgatcactt ggtgctggcc tgcacccgcg ctgagtttgg 5040
   ctctagcgat gaagatacag attgaggtac tgaaatgtgt gggcgtggct taagggtggg 5100
   aaagaatata taaggtgggg gtcttatgta gttttgtatc tgttttgcag cagccgccgc 5160
   cgccatgagc accaactcgt ttgatggaag cattgtgagc tcatatttga caacgcgcat 5220
   gcccccatgg gccggggtgc gtcagaatgt gatgggctcc agcattgatg gtcgccccgt 5280
   cctgcccgca aactctacta ccttgaccta cgagaccgtg tctggaacgc cgttggagac 5340
   tgcagcctcc gccgccgctt cagccgctgc agccaccgcc cgcgggattg tgactgactt 5400
   tgctttcctg agcccgcttg caagcagtgc agcttcccgt tcatccgccc gcgatgacaa 5460
   gttgacggct cttttggcac aattggattc tttgacccgg gaacttaatg tcgtttctca 5520
   gcagctgttg gatctgcgcc agcaggtttc tgccctgaag gcttcctccc ctcccaatgc 5580
   ggtttaaaac ataaataaaa aaccagactc tgtttggatt tggatcaagc aagtgtcttg 5640
   ctgtctttat ttaggggttt tgcgcgcgcg gtaggcccgg gaccagcggt ctcggtcgtt 5700
   gagggtcctg tgtatttttt ccaggacgtg gtaaaggtga ctctggatgt tcagatacat 5760
   gggcataagc ccgtctctgg ggtggaggta gcaccactgc agagcttcat gctgcggggt 5820
   ggtgttgtag atgatccagt cgtagcagga gcgctgggcg tggtgcctaa aaatgtcttt 5880
   cagtagcaag ctgattgcca ggggcaggcc cttggtgtaa gtgtttacaa agcggttaag 5940
   ctgggatggg tgcatacgtg gggatatgag atgcatcttg gactgtattt ttaggttggc 6000
   tatgttccca gccatatccc tccggggatt catgttgtgc agaaccacca gcacagtgta 6060
   tccggtgcac ttgggaaatt tgtcatgtag cttagaagga aatgcgtgga agaacttgga 6120
   gacgcccttg tgacctccaa gattttccat gcattcgtcc ataatgatgg caatgggccc 6180
   acgggcggcg gcctgggcga agatatttct gggatcacta acgtcatagt tgtgttccag 6240
   gatgagatcg tcataggcca tttttacaaa gcgcgggcgg agggtgccag actgcggtat 6300
   aatggttcca tccggcccag gggcgtagtt accctcacag atttgcattt cccacgcttt 6360
   gagttcagat ggggggatca tgtctacctg cggggcgatg aagaaaacgg tttccggggt 6420
   aggggagatc agctgggaag aaagcaggtt cctgagcagc tgcgacttac cgcagccggt 6480
   gggcccgtaa atcacaccta ttaccgggtg caactggtag ttaagagagc tgcagctgcc 6540
   gtcatccctg agcagggggg ccacttcgtt aagcatgtcc ctgactcgca tgttttccct 6600
   gaccaaatcc gccagaaggc gctcgccgcc cagcgatagc agttcttgca aggaagcaaa 6660
   gtttttcaac ggtttgagac cgtccgccgt aggcatgctt ttgagcgttt gaccaagcag   6720
   ttccaggcgg tcccacagct cggtcacctg ctctacggca tctcgatcca gcatatctcc   6780
   tcgtttcgcg ggttggggcg gctttcgctg tacggcagta gtcggtgctc gtccagacgg   6840
   gccagggtca tgtctttcca cgggcgcagg gtcctcgtca gcgtagtctg ggtcacggtg   6900
   aaggggtgcg ctccgggctg cgcgctggcc agggtgcgct tgaggctggt cctgctggtg   6960
   ctgaagcgct gccggtcttc gccctgcgcg tcggccaggt agcatttgac catggtgtca   7020
   tagtccagcc cctccgcggc gtggcccttg gcgcgcagct tgcccttgga ggaggcgccg   7080
   cacgaggggc agtgcagact tttgagggcg tagagcttgg gcgcgagaaa taccgattcc   7140
   ggggagtagg catccgcgcc gcaggccccg cagacggtct cgcattccac gagccaggtg   7200
   agctctggcc gttcggggtc aaaaaccagg tttcccccat gctttttgat gcgtttctta   7260
   cctctggttt ccatgagccg gtgtccacgc tcggtgacga aaaggctgtc cgtgtccccg   7320
   tatacagact tgagaggcct gtcctcgacc gatgcccttg agagccttca acccagtcag   7380
   ctccttccgg tgggcgcggg gcatgactat cgtcgccgca cttatgactg tcttctttat   7440
   catgcaactc gtaggacagg tgccggcagc gctctgggtc attttcggcg aggaccgctt   7500
   tcgctggagc gcgacgatga tcggcctgtc gcttgcggta ttcggaatct tgcacgccct   7560
   cgctcaagcc ttcgtcactg gtcccgccac caaacgtttc ggcgagaagc aggccattat   7620
   cgccggcatg gcggccgacg cgctgggcta cgtcttgctg gcgttcgcga cgcgaggctg   7680
   gatggccttc cccattatga ttcttctcgc ttccggcggc atcgggatgc ccgcgttgca   7740
   ggccatgctg tccaggcagg tagatgacga ccatcaggga cagcttcaag gatcgctcgc   7800
   ggctcttacc agcctaactt cgatcactgg accgctgatc gtcacggcga tttatgccgc   7860
   ctcggcgagc acatggaacg ggttggcatg gattgtaggc gccgccctat accttgtctg   7920
   cctccccgcg ttgcgtcgcg gtgcatggag ccgggccacc tcgacctgaa tggaagccgg   7980
   cggcacctcg ctaacggatt caccactcca agaattggag ccaatcaatt cttgcggaga   8040
   actgtgaatg cgcaaaccaa cccttggcag aacatatcca tcgcgtccgc catctccagc   8100
   agccgcacgc ggcgcatctc gggcagcgtt gggtcctggc cacgggtgcg catgatcgtg   8160
   ctcctgtcgt tgaggacccg gctaggctgg cggggttgcc ttactggtta gcagaatgaa   8220
   tcaccgatac gcgagcgaac gtgaagcgac tgctgctgca aaacgtctgc gacctgagca   8280
   acaacatgaa tggtcttcgg tttccgtgtt tcgtaaagtc tggaaacgcg gaagtcagcg   8340
   ccctgcacca ttatgttccg gatctgcatc gcaggatgct gctggctacc ctgtggaaca   8400
   cctacatctg tattaacgaa gcgctggcat tgaccctgag tgatttttct ctggtcccgc   8460
   cgcatccata ccgccagttg tttaccctca caacgttcca gtaaccgggc atgttcatca   8520
   tcagtaaccc gtatcgtgag catcctctct cgtttcatcg gtatcattac ccccatgaac   8580
   agaaattccc ccttacacgg aggcatcaag tgaccaaaca ggaaaaaacc gcccttaaca   8640
   tggcccgctt tatcagaagc cagacattaa cgcttctgga gaaactcaac gagctggacg   8700
   cggatgaaca ggcagacatc tgtgaatcgc ttcacgacca cgctgatgag ctttaccgca   8760
   gctgcctcgc gcgtttcggt gatgacggtg aaaacctctg acacatgcag ctcccggaga   8820
   cggtcacagc ttgtctgtaa gcggatgccg ggagcagaca agcccgtcag ggcgcgtcag   8880
   cgggtgttgg cgggtgtcgg ggcgcagcca tgacccagtc acgtagcgat agcggagtgt   8940
   atactggctt aactatgcgg catcagagca gattgtactg agagtgcacc atatgcggtg   9000
   tgaaataccg cacagatgcg taaggagaaa ataccgcatc aggcgctctt ccgcttcctc   9060
   gctcactgac tcgctgcgct cggtcgttcg gctgcggcga gcggtatcag ctcactcaaa   9120
   ggcggtaata cggttatcca cagaatcagg ggataacgca ggaaagaaca tgtgagcaaa   9180
   aggccagcaa aaggccagga accgtaaaaa ggccgcgttg ctggcgtttt tccataggct   9240
   ccgcccccct gacgagcatc acaaaaatcg acgctcaagt cagaggtggc gaaacccgac   9300
   aggactataa agataccagg cgtttccccc tggaagctcc ctcgtgcgct ctcctgttcc   9360
   gaccctgccg cttaccggat acctgtccgc ctttctccct tcgggaagcg tggcgctttc   9420
   tcaatgctca cgctgtaggt atctcagttc ggtgtaggtc gttcgctcca agctgggctg   9480
   tgtgcacgaa ccccccgttc agcccgaccg ctgcgcctta tccggtaact atcgtcttga   9540
   gtccaacccg gtaagacacg acttatcgcc actggcagca gccactggta acaggattag   9600
   cagagcgagg tatgtaggcg gtgctacaga gttcttgaag tggtggccta actacggcta   9660
   cactagaagg acagtatttg gtatctgcgc tctgctgaag ccagttacct tcggaaaaag   9720
   agttggtagc tcttgatccg gcaaacaaac caccgctggt agcggtggtt tttttgtttg   9780
   caagcagcag attacgcgca gaaaaaaagg atctcaagaa gatcctttga tcttttctac   9840
   ggggtctgac gctcagtgga acgaaaactc acgttaaggg attttggtca tgagattatc   9900
   aaaaaggatc ttcacctaga tccttttaaa ttaaaaatga agttttaaat caatctaaag   9960
   tatatatgag taaacttggt ctgacagtta ccaatgctta atcagtgagg cacctatctc  10020
   agcgatctgt ctatttcgtt catccatagt tgcctgactc cccgtcgtgt agataactac  10080
   gatacgggag ggcttaccat ctggccccag tgctgcaatg ataccgcgag acccacgctc  10140
   accggctcca gatttatcag caataaacca gccagccgga agggccgagc gcagaagtgg  10200
   tcctgcaact ttatccgcct ccatccagtc tattaattgt tgccgggaag ctagagtaag  10260
   tagttcgcca gttaatagtt tgcgcaacgt tgttgccatt gctgcaggca tcgtggtgtc  10320
   acgctcgtcg tttggtatgg cttcattcag ctccggttcc caacgatcaa ggcgagttac   10380
   atgatccccc atgttgtgca aaaaagcggt tagctccttc ggtcctccga tcgttgtcag   10440
   aagtaagttg gccgcagtgt tatcactcat ggttatggca gcactgcata attctcttac   10500
   tgtcatgcca tccgtaagat gcttttctgt gactggtgag tactcaacca agtcattctg   10560
   agaatagtgt atgcggcgac cgagttgctc ttgcccggcg tcaacacggg ataataccgc   10620
   gccacatagc agaactttaa aagtgctcat cattggaaaa cgttcttcgg ggcgaaaact   10680
   ctcaaggatc ttaccgctgt tgagatccag ttcgatgtaa cccactcgtg cacccaactg   10740
   atcttcagca tcttttactt tcaccagcgt ttctgggtga gcaaaaacag gaaggcaaaa   10800
   tgccgcaaaa aagggaataa gggcgacacg gaaatgttga atactcatac tcttcctttt   10860
   tcaatattat tgaagcattt atcagggtta ttgtctcatg agcggataca tatttgaatg   10920
   tatttagaaa aataaacaaa taggggttcc gcgcacattt ccccgaaaag tgccacctga   10980
   cgtctaagaa accattatta tcatgacatt aacctataaa aataggcgta tcacgaggcc   11040
   ctttcgtctt caagaattct tatcatgaca ttaacctata aaaataggcg tatcacgagg   11100
   ccctttcgtc atcatcaata atatacctta ttttggattg aagccaatat gataatgagg   11160
   gggtggagtt tgtgacgtgg cgcggggcgt gggaacgggg cgggtgacgt agtagtgtgg   11220
   cggaagtgtg atgttgcaag tgtggcggaa cacatgtaag cgccggatgt ggtaaaagtg   11280
   acgtttttgg tgtgcgccgg tgtatacggg aagtgacaat tttcgcgcgg ttttaggcgg   11340
   atgttgtagt aaatttgggc gtaaccaagt aatgtttggc cattttcgcg ggaaaactga   11400
   ataagaggaa gtgaaatctg aataattctg tgttactcat agcgcgtaat atttgtctag   11460
   ggccgcgggg actttgaccg tttacgtgga gactcgccca ggtgtttttc tcaggtgttt   11520
   tccgcgttcc gggtcaaagt tggcgtttta ttattatagt cagctctag               11569
<210> 2
<211> 11458
   <212> DNA
   <213> Artificial Sequence
<220>
   <223> Adenovirales Expressionsplasmid pAd.3r.mscIL-12 zur regulierten Expression des murinen IL-12
<220>
   <221> gene
   <222> (327)..(713)
   <223> VP16
<220>
   <221> gene
   <222> (714)..(1352)
   <223> TetR
<220>
   <221> Intron
   <222> (1353)..(1912)
   <223>
<220>
   <221> promoter
   <222> (1864)..(1902)
   <223> TK-min
<220>
   <221> protein_bind
   <222> (1913)..(2212)
   <223> Tet07
<220>
   <221> misc_feature
   <222> (2213)..(2687)
   <223> CMV-min + Intron
<220>
   <221> promoter
   <222> (2226)..(2264)
   <223> CMV-min
<220>
   <221> gene
   <222> (2688)..(4325)
   <223> Murines single-chain IL-12
<400> 2
   ctgctggttc tttccgcctc agaagccata gagcccaccg catccccagc atgcctgcta     60
   ttgtcttccc aatcctcccc cttgctgtcc tgccccaccc caccccccag aatagaatga    120
   cacctactca gacaatgcga tgcaatttcc tcattttatt aggaaaggac agtgggagtg    180
   gcaccttcca gggtcaagga aggcacgggg gaggggcaaa caacagatgg ctggcaacta    240
   gaaggcacag tcgaggctga tcagcgagct ctagcattta ggtgacacta tagaataggg    300
   ccctctagga tcgatcctcg cgccccctac ccaccgtact cgtcaattcc aagggcatcg    360
   gtaaacatct gctcaaactc gaagtcggcc atatccagag cgccgtaggg ggcggagtcg    420
   tggggggtaa atcccggacc cggggaatcc ccgtccccca acatgtccag atcgaaatcg    480
   tctagcgcgt cggcatgcgc catcgccacg tcctcgccgt ctaagtggag ctcgtccccc    540
   aggctgacat cggtcggggg ggccgtggac agtctgcgcg tgtgtcccgc ggggagaaag    600
   gacaggcgcg gagccgccag ccccgcctct tcgggggcgt cgtcgtccgg gagatcgagc    660
   aggccctcga tggtagaccc gtaattgttt ttcgtacgcg cgcggctgta cgcggaccca    720
   ctttcacatt taagttgttt ttctaatccg catatgatca attcaaggcc gaataagaag    780
   gctggctctg caccttggtg atcaaataat tcgatagctt gtcgtaataa tggcggcata    840
   ctatcagtag taggtgtttc cctttcttct ttagcgactt gatgctcttg atcttccaat    900
   acgcaaccta aagtaaaatg ccccacagcg ctgagtgcat ataatgcatt ctctagtgaa    960
   aaaccttgtt ggcataaaaa ggctaattga ttttcgagag tttcatactg tttttctgta   1020
   ggccgtgtac ctaaatgtac ttttgctcca tcgcgatgac ttagtaaagc acatctaaaa   1080
   cttttagcgt tattacgtaa aaaatcttgc cagctttccc cttctaaagg gcaaaagtga   1140
   gtatggtgcc tatctaacat ctcaatggct aaggcgtcga gcaaagcccg cttatttttt   1200
   acatgccaat acaatgtagg ctgctctaca cctagcttct gggcgagttt acgggttgtt   1260
   aaaccttcga ttccgacctc attaagcagc tctaatgcgc tgttaatcac tttactttta   1320
   tctaatctag agggtctggg tctctttggc atggtcgaat taattcgcgt cgagccggcc   1380
   gcgggtacaa ttccggttgg acctgggagt ggacacctgt ggagagaaag gcaaagtgga   1440
   tgtcattgtc actcaagtgt atggccagat ctcaagcctg ccacacctca agcttgacaa   1500
   caaaaagatt gtcttttctg accagatgga cgcggccacc ctcaaaggca tcaccgcggg   1560
   ccaggtgaat atcaaatcct cctcgttttt ggaaactgac aatcttagcg cagaagtcat   1620
   gcccgctttt gagagggagt actcacccca acagtcgaga ggttttccga tccggtcgat   1680
   gcggactcgc tcaggtccct cggtggcgga gtaccgttcg gaggccgacg ggtttccgat   1740
   ccaagagtac tggaaagacc gcgaagagtt tgtcctcaac cgcgagccca acaggcgtcg   1800
   aagcttgatg ggtcgctcgg tgttcgaggc cacacgcgtc accttaatat gcgaagtgga   1860
   cctcggaccg cgccgccccg actgcatctg cgtgttcgaa ttgcccggcg agctcgactt   1920
   tcacttttct ctatcactga tagggagtgg taaactcgac tttcactttt ctctatcact   1980
   gatagggagt ggtaaactcg actttcactt ttctctatca ctgataggga gtggtaaact   2040
   cgactttcac ttttctctat cactgatagg gagtggtaaa ctcgactttc acttttctct   2100
   atcactgata gggagtggta aactcgactt tcacttttct ctatcactga tagggagtgg   2160
   taaactcgac tttcactttt ctctatcact gatagggagt ggtaaactcg acggtcgagg   2220
   gtcgagtagg cgtgtacggt gggaggccta tataagcaga gctcgtttag tgaaccgtca   2280
   gatcgcctgg agacgccatc cacgctgttt tgacctccat agaagacacc gggaccgatc   2340
   cagcctccgc ggccccgaat tgcgaagctt tattgcggta gtttatcaca gttaaattgc   2400
   taacgcagtc agtgcttctg acacaacagt ctcgaactta agctgcagaa gttggtcgtg   2460
   aggcactggg caggtaagta tcaaggttac aagacaggtt taaggagacc aatagaaact   2520
   gggcttgtcg agacagagaa gactcttgcg tttctgatag gcacctattg gtcttactga   2580
   catccacttt gcctttctct ccacaggtgt ccactcccag ttcaattaca gctcttaagg   2640
   ctagagtact taatacgact cactataggc tagcctcgag aattcgaatg gccatgggtc   2700
   ctcagaagct aaccatctcc tggtttgcca tcgttttgct ggtgtctcca ctcatggcca   2760
   tgtgggagct ggagaaagac gtttatgttg tagaggtgga ctggactccc gatgcccctg   2820
   gagaaacagt gaacctcacc tgtgacacgc ctgaagaaga tgacatcacc tggacctcag 2880
   accagagaca tggagtcata ggctctggaa agaccctgac catcactgtc aaagagtttc 2940
   tagatgctgg ccagtacacc tgccacaaag gaggcgagac tctgagccac tcacatctgc 3000
   tgctccacaa gaaggaaaat ggaatttggt ccactgaaat tttaaaaaat ttcaaaaaca 3060
   agactttcct gaagtgtgaa gcaccaaatt actccggacg gttcacgtgc tcatggctgg 3120
   tgcaaagaaa catggacttg aagttcaaca tcaagagcag tagcagttcc cctgactctc 3180
   gggcagtgac atgtggaatg gcgtctctgt ctgcagagaa ggtcacactg gaccaaaggg 3240
   actatgagaa gtattcagtg tcctgccagg aggatgtcac ctgcccaact gccgaggaga 3300
   ccctgcccat tgaactggcg ttggaagcac ggcagcagaa taaatatgag aactacagca 3360
   ccagcttctt catcagggac atcatcaaac cagacccgcc caagaacttg cagatgaagc 3420
   ctttgaagaa ctcacaggtg gaggtcagct gggagtaccc tgactcctgg agcactcccc 3480
   attcctactt ctccctcaag ttctttgttc gaatccagcg caagaaagaa aagatgaagg 3540
   agacagagga ggggtgtaac cagaaaggtg cgttcctcgt agagaagaca tctaccgaag 3600
   tccaatgcaa aggcgggaat gtctgcgtgc aagctcagga tcgctattac aattcctcat 3660
   gcagcaagtg ggcatgtgtt ccctgcaggg tccgatccgg tggcggtggc tcgggcggtg 3720
   gtgggtcggg tggcggcgga tctagggtca ttccagtctc tggacctgcc aggtgtctta 3780
   gccagtcccg aaacctgctg aagaccacag atgacatggt gaagacggcc agagaaaaac 3840
   tgaaacatta ttcctgcact gctgaagaca tcgatcatga agacatcaca cgggaccaaa 3900
   ccagcacatt gaagacctgt ttaccactgg aactacacaa gaacgagagt tgcctggcta 3960
   ctagagagac ttcttccaca acaagaggga gctgcctgcc cccacagaag acgtctttga 4020
   tgatgaccct gtgccttggt agcatctatg aggacttgaa gatgtaccag acagagttcc 4080
   aggccatcaa cgcagcactt cagaatcaca accatcagca gatcattcta gacaagggca 4140
   tgctggtggc catcgatgag ctgatgcagt ctctgaatca taatggcgag actctgcgcc 4200
   agaaacctcc tgtgggagaa gcagaccctt acagagtgaa aatgaagctc tgcatcctgc 4260
   ttcacgcctt cagcacccgc gtcgtgacca tcaacagggt gatgggctat ctgagctccg 4320
   cctgagaatt gatccggatt agtccaattt gttaaagaca ggatgggccc tatatatgga 4380
   tccactagta acggccgcca gtgtgctgga attctgcaga tatccatcac actggcggcc 4440
   gctcgagcat gcatctagag ggccctattc tatagtgtca cctaaatgct agagctcgct 4500
   gatcagcctc gactgtgcct tctagttgcc agccatctgt tgtttgcccc tcccccgtgc 4560
   cttccttgac cctggaaggt gccactccca ctgtcctttc ctaataaaat gaggaaattg 4620
   catcgcattg tctgagtagg tgtcattcta ttctgggggg tggggtgggg caggacagca 4680
   agggggagga ttgggaagac aatagcaggc atgctgggga tgcggtgggc tctatggctt 4740
   ctgaggcgga aagaaccagt cgacatcgat gctagagtgg aaggtgctga ggtacgatga 4800
   gacccgcacc aggtgcagac cctgcgagtg tggcggtaaa catattagga accagcctgt 4860
   gatgctggat gtgaccgagg agctgaggcc cgatcacttg gtgctggcct gcacccgcgc 4920
   tgagtttggc tctagcgatg aagatacaga ttgaggtact gaaatgtgtg ggcgtggctt 4980
   aagggtggga aagaatatat aaggtggggg tcttatgtag ttttgtatct gttttgcagc 5040
   agccgccgcc gccatgagca ccaactcgtt tgatggaagc attgtgagct catatttgac 5100
   aacgcgcatg cccccatggg ccggggtgcg tcagaatgtg atgggctcca gcattgatgg 5160
   tcgccccgtc ctgcccgcaa actctactac cttgacctac gagaccgtgt ctggaacgcc 5220
   gttggagact gcagcctccg ccgccgcttc agccgctgca gccaccgccc gcgggattgt 5280
   gactgacttt gctttcctga gcccgcttgc aagcagtgca gcttcccgtt catccgcccg 5340
   cgatgacaag ttgacggctc ttttggcaca attggattct ttgacccggg aacttaatgt 5400
   cgtttctcag cagctgttgg atctgcgcca gcaggtttct gccctgaagg cttcctcccc 5460
   tcccaatgcg gtttaaaaca taaataaaaa accagactct gtttggattt ggatcaagca 5520
   agtgtcttgc tgtctttatt taggggtttt gcgcgcgcgg taggcccggg accagcggtc 5580
   tcggtcgttg agggtcctgt gtattttttc caggacgtgg taaaggtgac tctggatgtt 5640
   cagatacatg ggcataagcc cgtctctggg gtggaggtag caccactgca gagcttcatg 5700
   ctgcggggtg gtgttgtaga tgatccagtc gtagcaggag cgctgggcgt ggtgcctaaa 5760
   aatgtctttc agtagcaagc tgattgccag gggcaggccc ttggtgtaag tgtttacaaa 5820
   gcggttaagc tgggatgggt gcatacgtgg ggatatgaga tgcatcttgg actgtatttt 5880
   taggttggct atgttcccag ccatatccct ccggggattc atgttgtgca gaaccaccag 5940
   cacagtgtat ccggtgcact tgggaaattt gtcatgtagc ttagaaggaa atgcgtggaa 6000
   gaacttggag acgcccttgt gacctccaag attttccatg cattcgtcca taatgatggc 6060
   aatgggccca cgggcggcgg cctgggcgaa gatatttctg ggatcactaa cgtcatagtt 6120
   gtgttccagg atgagatcgt cataggccat ttttacaaag cgcgggcgga gggtgccaga 6180
   ctgcggtata atggttccat ccggcccagg ggcgtagtta ccctcacaga tttgcatttc 6240
   ccacgctttg agttcagatg gggggatcat gtctacctgc ggggcgatga agaaaacggt 6300
   ttccggggta ggggagatca gctgggaaga aagcaggttc ctgagcagct gcgacttacc 6360
   gcagccggtg ggcccgtaaa tcacacctat taccgggtgc aactggtagt taagagagct 6420
   gcagctgccg tcatccctga gcaggggggc cacttcgtta agcatgtccc tgactcgcat 6480
   gttttccctg accaaatccg ccagaaggcg ctcgccgccc agcgatagca gttcttgcaa   6540
   ggaagcaaag tttttcaacg gtttgagacc gtccgccgta ggcatgcttt tgagcgtttg   6600
   accaagcagt tccaggcggt cccacagctc ggtcacctgc tctacggcat ctcgatccag   6660
   catatctcct cgtttcgcgg gttggggcgg ctttcgctgt acggcagtag tcggtgctcg   6720
   tccagacggg ccagggtcat gtctttccac gggcgcaggg tcctcgtcag cgtagtctgg   6780
   gtcacggtga aggggtgcgc tccgggctgc gcgctggcca gggtgcgctt gaggctggtc   6840
   ctgctggtgc tgaagcgctg ccggtcttcg ccctgcgcgt cggccaggta gcatttgacc   6900
   atggtgtcat agtccagccc ctccgcggcg tggcccttgg cgcgcagctt gcccttggag   6960
   gaggcgccgc acgaggggca gtgcagactt ttgagggcgt agagcttggg cgcgagaaat   7020
   accgattccg gggagtaggc atccgcgccg caggccccgc agacggtctc gcattccacg   7080
   agccaggtga gctctggccg ttcggggtca aaaaccaggt ttcccccatg ctttttgatg   7140
   cgtttcttac ctctggtttc catgagccgg tgtccacgct cggtgacgaa aaggctgtcc   7200
   gtgtccccgt atacagactt gagaggcctg tcctcgaccg atgcccttga gagccttcaa   7260
   cccagtcagc tccttccggt gggcgcgggg catgactatc gtcgccgcac ttatgactgt   7320
   cttctttatc atgcaactcg taggacaggt gccggcagcg ctctgggtca ttttcggcga   7380
   ggaccgcttt cgctggagcg cgacgatgat cggcctgtcg cttgcggtat tcggaatctt   7440
   gcacgccctc gctcaagcct tcgtcactgg tcccgccacc aaacgtttcg gcgagaagca   7500
   ggccattatc gccggcatgg cggccgacgc gctgggctac gtcttgctgg cgttcgcgac   7560
   gcgaggctgg atggccttcc ccattatgat tcttctcgct tccggcggca tcgggatgcc   7620
   cgcgttgcag gccatgctgt ccaggcaggt agatgacgac catcagggac agcttcaagg   7680
   atcgctcgcg gctcttacca gcctaacttc gatcactgga ccgctgatcg tcacggcgat   7740
   ttatgccgcc tcggcgagca catggaacgg gttggcatgg attgtaggcg ccgccctata   7800
   ccttgtctgc ctccccgcgt tgcgtcgcgg tgcatggagc cgggccacct cgacctgaat   7860
   ggaagccggc ggcacctcgc taacggattc accactccaa gaattggagc caatcaattc   7920
   ttgcggagaa ctgtgaatgc gcaaaccaac ccttggcaga acatatccat cgcgtccgcc   7980
   atctccagca gccgcacgcg gcgcatctcg ggcagcgttg ggtcctggcc acgggtgcgc   8040
   atgatcgtgc tcctgtcgtt gaggacccgg ctaggctggc ggggttgcct tactggttag   8100
   cagaatgaat caccgatacg cgagcgaacg tgaagcgact gctgctgcaa aacgtctgcg   8160
   acctgagcaa caacatgaat ggtcttcggt ttccgtgttt cgtaaagtct ggaaacgcgg   8220
   aagtcagcgc cctgcaccat tatgttccgg atctgcatcg caggatgctg ctggctaccc   8280
   tgtggaacac ctacatctgt attaacgaag cgctggcatt gaccctgagt gatttttctc   8340
   tggtcccgcc gcatccatac cgccagttgt ttaccctcac aacgttccag taaccgggca   8400
   tgttcatcat cagtaacccg tatcgtgagc atcctctctc gtttcatcgg tatcattacc   8460
   cccatgaaca gaaattcccc cttacacgga ggcatcaagt gaccaaacag gaaaaaaccg   8520
   cccttaacat ggcccgcttt atcagaagcc agacattaac gcttctggag aaactcaacg   8580
   agctggacgc ggatgaacag gcagacatct gtgaatcgct tcacgaccac gctgatgagc   8640
   tttaccgcag ctgcctcgcg cgtttcggtg atgacggtga aaacctctga cacatgcagc   8700
   tcccggagac ggtcacagct tgtctgtaag cggatgccgg gagcagacaa gcccgtcagg   8760
   gcgcgtcagc gggtgttggc gggtgtcggg gcgcagccat gacccagtca cgtagcgata   8820
   gcggagtgta tactggctta actatgcggc atcagagcag attgtactga gagtgcacca   8880
   tatgcggtgt gaaataccgc acagatgcgt aaggagaaaa taccgcatca ggcgctcttc   8940
   cgcttcctcg ctcactgact cgctgcgctc ggtcgttcgg ctgcggcgag cggtatcagc   9000
   tcactcaaag gcggtaatac ggttatccac agaatcaggg gataacgcag gaaagaacat   9060
   gtgagcaaaa ggccagcaaa aggccaggaa ccgtaaaaag gccgcgttgc tggcgttttt   9120
   ccataggctc cgcccccctg acgagcatca caaaaatcga cgctcaagtc agaggtggcg   9180
   aaacccgaca ggactataaa gataccaggc gtttccccct ggaagctccc tcgtgcgctc   9240
   tcctgttccg accctgccgc ttaccggata cctgtccgcc tttctccctt cgggaagcgt   9300
   ggcgctttct caatgctcac gctgtaggta tctcagttcg gtgtaggtcg ttcgctccaa   9360
   gctgggctgt gtgcacgaac cccccgttca gcccgaccgc tgcgccttat ccggtaacta   9420
   tcgtcttgag tccaacccgg taagacacga cttatcgcca ctggcagcag ccactggtaa   9480
   caggattagc agagcgaggt atgtaggcgg tgctacagag ttcttgaagt ggtggcctaa   9540
   ctacggctac actagaagga cagtatttgg tatctgcgct ctgctgaagc cagttacctt   9600
   cggaaaaaga gttggtagct cttgatccgg caaacaaacc accgctggta gcggtggttt   9660
   ttttgtttgc aagcagcaga ttacgcgcag aaaaaaagga tctcaagaag atcctttgat   9720
   cttttctacg gggtctgacg ctcagtggaa cgaaaactca cgttaaggga ttttggtcat   9780
   gagattatca aaaaggatct tcacctagat ccttttaaat taaaaatgaa gttttaaatc   9840
   aatctaaagt atatatgagt aaacttggtc tgacagttac caatgcttaa tcagtgaggc   9900
   acctatctca gcgatctgtc tatttcgttc atccatagtt gcctgactcc ccgtcgtgta   9960
   gataactacg atacgggagg gcttaccatc tggccccagt gctgcaatga taccgcgaga   10020
   cccacgctca ccggctccag atttatcagc aataaaccag ccagccggaa gggccgagcg   10080
   cagaagtggt cctgcaactt tatccgcctc catccagtct attaattgtt gccgggaagc   10140
   tagagtaagt agttcgccag ttaatagttt gcgcaacgtt gttgccattg ctgcaggcat   10200
   cgtggtgtca cgctcgtcgt ttggtatggc ttcattcagc tccggttccc aacgatcaag   10260
   gcgagttaca tgatccccca tgttgtgcaa aaaagcggtt agctccttcg gtcctccgat   10320
   cgttgtcaga agtaagttgg ccgcagtgtt atcactcatg gttatggcag cactgcataa   10380
   ttctcttact gtcatgccat ccgtaagatg cttttctgtg actggtgagt actcaaccaa   10440
   gtcattctga gaatagtgta tgcggcgacc gagttgctct tgcccggcgt caacacggga   10500
   taataccgcg ccacatagca gaactttaaa agtgctcatc attggaaaac gttcttcggg   10560
   gcgaaaactc tcaaggatct taccgctgtt gagatccagt tcgatgtaac ccactcgtgc   10620
   acccaactga tcttcagcat cttttacttt caccagcgtt tctgggtgag caaaaacagg   10680
   aaggcaaaat gccgcaaaaa agggaataag ggcgacacgg aaatgttgaa tactcatact   10740
   cttccttttt caatattatt gaagcattta tcagggttat tgtctcatga gcggatacat   10800
   atttgaatgt atttagaaaa ataaacaaat aggggttccg cgcacatttc cccgaaaagt   10860
   gccacctgac gtctaagaaa ccattattat catgacatta acctataaaa ataggcgtat   10920
   cacgaggccc tttcgtcttc aagaattctt atcatgacat taacctataa aaataggcgt   10980
   atcacgaggc cctttcgtca tcatcaataa tataccttat tttggattga agccaatatg   11040
   ataatgaggg ggtggagttt gtgacgtggc gcggggcgtg ggaacggggc gggtgacgta   11100
   gtagtgtggc ggaagtgtga tgttgcaagt gtggcggaac acatgtaagc gccggatgtg   11160
   gtaaaagtga cgtttttggt gtgcgccggt gtatacggga agtgacaatt ttcgcgcggt   11220
   tttaggcgga tgttgtagta aatttgggcg taaccaagta atgtttggcc attttcgcgg   11280
   gaaaactgaa taagaggaag tgaaatctga ataattctgt gttactcata gcgcgtaata   11340
   tttgtctagg gccgcgggga ctttgaccgt ttacgtggag actcgcccag gtgtttttct   11400
   caggtgtttt ccgcgttccg ggtcaaagtt ggcgttttat tattatagtc agctctag     11458
<210> 3
   <211> 11453
   <212> DNA
   <213> Artificial Sequence
<220>
   <223> Adenovirales Expressionsplasmid pShuttle.3r.hscIL-12 zur reguliertenExpression des humanen IL-12 nach Virusgeneration mittels AdEasy
<220>
   <221> gene
   <222> (327)..(713)
   <223> VP16
<220>
   <221> gene
   <222> (714)..(1352)
   <223> TetR
<220>
   <221> Intron
   <222> (1353)..(1912)
   <223>
<220>
   <221> promoter
   <222> (1864)..(1902)
   <223> TK-min
<220>
   <221> protein_bind
   <222> (1913) .. (2212)
   <223> TetO7
<220>
   <221> misc_feature
   <222> (2213)..(2709)
   <223> CMV-min + Intron
<220>
   <221> promoter
   <222> (2226)..(2264)
   <223> CMV-min
<220>
   <221> gene
   <222> (2710)..(4308)
   <223> Humanes singe-chain IL-12
<220>
   <221> mutation
   <222> (4020)..(4023)
   <223> T/C, A/T, G/C, T/G
<400> 3
   ctgctggttc tttccgcctc agaagccata gagcccaccg catccccagc atgcctgcta     60
   ttgtcttccc aatcctcccc cttgctgtcc tgccccaccc caccccccag aatagaatga     120
   cacctactca gacaatgcga tgcaatttcc tcattttatt aggaaaggac agtgggagtg    180
   gcaccttcca gggtcaagga aggcacgggg gaggggcaaa caacagatgg ctggcaacta    240
   gaaggcacag tcgaggctga tcagcgagct ctagcattta ggtgacacta tagaataggg    300
   ccctctagga tcgatcctcg cgccccctac ccaccgtact cgtcaattcc aagggcatcg    360
   gtaaacatct gctcaaactc gaagtcggcc atatccagag cgccgtaggg ggcggagtcg    420
   tggggggtaa atcccggacc cggggaatcc ccgtccccca acatgtccag atcgaaatcg    480
   tctagcgcgt cggcatgcgc catcgccacg tcctcgccgt ctaagtggag ctcgtccccc    540
   aggctgacat cggtcggggg ggccgtggac agtctgcgcg tgtgtcccgc ggggagaaag    600
   gacaggcgcg gagccgccag ccccgcctct tcgggggcgt cgtcgtccgg gagatcgagc    660
   aggccctcga tggtagaccc gtaattgttt ttcgtacgcg cgcggctgta cgcggaccca    720
   ctttcacatt taagttgttt ttctaatccg catatgatca attcaaggcc gaataagaag    780
   gctggctctg caccttggtg atcaaataat tcgatagctt gtcgtaataa tggcggcata    840
   ctatcagtag taggtgtttc cctttcttct ttagcgactt gatgctcttg atcttccaat    900
   acgcaaccta aagtaaaatg ccccacagcg ctgagtgcat ataatgcatt ctctagtgaa    960
   aaaccttgtt ggcataaaaa ggctaattga ttttcgagag tttcatactg tttttctgta   1020
   ggccgtgtac ctaaatgtac ttttgctcca tcgcgatgac ttagtaaagc acatctaaaa   1080
   cttttagcgt tattacgtaa aaaatcttgc cagctttccc cttctaaagg gcaaaagtga   1140
   gtatggtgcc tatctaacat ctcaatggct aaggcgtcga gcaaagcccg cttatttttt   1200
   acatgccaat acaatgtagg ctgctctaca cctagcttct gggcgagttt acgggttgtt   1260
   aaaccttcga ttccgacctc attaagcagc tctaatgcgc tgttaatcac tttactttta   1320
   tctaatctag agggtctggg tctctttggc atggtcgaat taattcgcgt cgagccggcc   1380
   gcgggtacaa ttccggttgg acctgggagt ggacacctgt ggagagaaag gcaaagtgga   1440
   tgtcattgtc actcaagtgt atggccagat ctcaagcctg ccacacctca agcttgacaa   1500
   caaaaagatt gtcttttctg accagatgga cgcggccacc ctcaaaggca tcaccgcggg   1560
   ccaggtgaat atcaaatcct cctcgttttt ggaaactgac aatcttagcg cagaagtcat   1620
   gcccgctttt gagagggagt actcacccca acagtcgaga ggttttccga tccggtcgat   1680
   gcggactcgc tcaggtccct cggtggcgga gtaccgttcg gaggccgacg ggtttccgat   1740
   ccaagagtac tggaaagacc gcgaagagtt tgtcctcaac cgcgagccca acaggcgtcg   1800
   aagcttgatg ggtcgctcgg tgttcgaggc cacacgcgtc accttaatat gcgaagtgga   1860
   cctcggaccg cgccgccccg actgcatctg cgtgttcgaa ttgcccggcg agctcgactt   1920
   tcacttttct ctatcactga tagggagtgg taaactcgac tttcactttt ctctatcact   1980
   gatagggagt ggtaaactcg actttcactt ttctctatca ctgataggga gtggtaaact   2040
   cgactttcac ttttctctat cactgatagg gagtggtaaa ctcgactttc acttttctct   2100
   atcactgata gggagtggta aactcgactt tcacttttct ctatcactga tagggagtgg   2160
   taaactcgac tttcactttt ctctatcact gatagggagt ggtaaactcg acggtcgagg   2220
   gtcgagtagg cgtgtacggt gggaggccta tataagcaga gctcgtttag tgaaccgtca   2280
   gatcgcctgg agacgccatc cacgctgttt tgacctccat agaagacacc gggaccgatc   2340
   cagcctccgc ggccccgaat tgcgaagctt tattgcggta gtttatcaca gttaaattgc   2400
   taacgcagtc agtgcttctg acacaacagt ctcgaactta agctgcagaa gttggtcgtg   2460
   aggcactggg caggtaagta tcaaggttac aagacaggtt taaggagacc aatagaaact   2520
   gggcttgtcg agacagagaa gactcttgcg tttctgatag gcacctattg gtcttactga   2580
   catccacttt gcctttctct ccacaggtgt ccactcccag ttcaattaca gctcttaagg    2640
   ctagagtact taatacgact cactataggc tagcctcgag aattcacgcg tggtaccgag    2700
   ctcggatcca tgggtcacca gcagttggtc atctcttggt tttccctggt ttttctggca    2760
   tctcccctcg tggccatatg ggaactgaag aaagatgttt atgtcgtaga attggattgg    2820
   tatccggatg cccctggaga aatggtggtc ctcacctgtg acacccctga agaagatggt    2880
   atcacctgga ccttggacca gagcagtgag gtcttaggct ctggcaaaac cctgaccatc    2940
   caagtcaaag agtttggaga tgctggccag tacacctgtc acaaaggagg cgaggttcta    3000
   agccattcgc tcctgctgct tcacaaaaag gaagatggaa tttggtccac tgatatttta    3060
   aaggaccaga aagaacccaa aaataagacc tttctaagat gcgaggccaa gaattattct    3120
   ggacgtttca cctgctggtg gctgacgaca atcagtactg atttgacatt cagtgtcaaa    3180
   agcagcagag gctcttctga cccccaaggg gtgacgtgcg gagctgctac actctctgca    3240
   gagagagtca gaggggacaa caaggagtat gagtactcag tggagtgcca ggaggacagt    3300
   gcctgcccag ctgctgagga gagtctgccc attgaggtca tggtggatgc cgttcacaag    3360
   ctcaagtatg aaaactacac cagcagcttc ttcatcaggg acatcatcaa acctgaccca    3420
   cccaacaact tgcagctgaa gccattaaag aattctcggc aggtggaggt cagctgggag    3480
   taccctgaca cctggagtac tccacattcc tacttctccc tgacattctg cgttcaggtc    3540
   cagggcaaga gcaagagaga aaagaaagat agagtcttca cggacaagac ctcagccacg    3600
   gtcatctgcc gcaaaaatgc cagcattagc gtgcgggccc aggaccgcta ctatagctca    3660
   tcttggagcg aatgggcatc tgtgccctgc agtggtggcg gtggcggcgg atctagaaac    3720
   ctccccgtgg ccactccaga cccaggaatg ttcccatgcc ttcaccactc ccaaaacctg    3780
   ctgagggccg tcagcaacat gctccagaag gccagacaaa ctctagaatt ttacccttgc    3840
   acttctgaag agattgatca tgaagatatc acaaaagata aaaccagcac agtggaggcc    3900
   tgtttaccat tggaattaac caagaatgag agttgcctaa attccagaga gacctctttc    3960
   ataactaatg ggagttgcct ggcctccaga aagacctctt ttatgatggc cctgtgcctc    4020
   tcgagtattt atgaagactc gaagatgtac caggtggagt tcaagaccat gaatgcaaag    4080
   cttctgatgg atcctaagag gcagatcttt ctagatcaaa acatgctggc agttattgat    4140
   gagctgatgc aggccctgaa tttcaacagt gagactgtgc cacaaaaatc ctcccttgaa    4200
   gaaccggatt tttataaaac taaaatcaag ctctgcatac ttcttcatgc tttcagaatt    4260
   cgggcagtga ctattgatag agtgatgagc tatctgaatg cttcctaaaa agcgaggtcg    4320
   atccggatta gtccaatttg ttaaagacag gatatcagtg gtccaggctc tagttttgac    4380
   tcaacaatat caccagctga agcctataga gtacgagcca tagataaaat aaaagatttt    4440
   atttagtctc cagaaaaagg ggggaatgaa agaccccacc tgtaggtttg gcaagctagt    4500
   aacggccgcc agtgtgctgg aattctgcag atatccatca cactggcggc cgctcgagca    4560
   tgcatctaga gggccctatt ctatagtgtc acctaaatgc tagagctcgc tgatcagcct    4620
   cgactgtgcc ttctagttgc cagccatctg ttgtttgccc ctcccccgtg ccttccttga    4680
   ccctggaagg tgccactccc actgtccttt cctaataaaa tgaggaaatt gcatcgcatt    4740
   gtctgagtag gtgtcattct attctggggg gtggggtggg gcaggacagc aagggggagg    4800
   attgggaaga caatagcagg catgctgggg atgcggtggg ctctatggct tctgaggcgg    4860
   aaagaaccag tcgactcgaa gatctgggcg tggttaaggg tgggaaagaa tatataaggt    4920
   gggggtctta tgtagttttg tatctgtttt gcagcagccg ccgccgccat gagcaccaac    4980
   tcgtttgatg gaagcattgt gagctcatat ttgacaacgc gcatgccccc atgggccggg    5040
   gtgcgtcaga atgtgatggg ctccagcatt gatggtcgcc ccgtcctgcc cgcaaactct    5100
   actaccttga cctacgagac cgtgtctgga acgccgttgg agactgcagc ctccgccgcc    5160
   gcttcagccg ctgcagccac cgcccgcggg attgtgactg actttgcttt cctgagcccg    5220
   cttgcaagca gtgcagcttc ccgttcatcc gcccgcgatg acaagttgac ggctcttttg    5280
   gcacaattgg attctttgac ccgggaactt aatgtcgttt ctcagcagct gttggatctg    5340
   cgccagcagg tttctgccct gaaggcttcc tcccctccca atgcggttta aaacataaat    5400
   aaaaaaccag actctgtttg gatttggatc aagcaagtgt cttgctgtct ttatttaggg    5460
   gttttgcgcg cgcggtaggc ccgggaccag cggtctcggt cgttgagggt cctgtgtatt    5520
   ttttccagga cgtggtaaag gtgactctgg atgttcagat acatgggcat aagcccgtct    5580
   ctggggtgga ggtagcacca ctgcagagct tcatgctgcg gggtggtgtt gtagatgatc    5640
   cagtcgtagc aggagcgctg ggcgtggtgc ctaaaaatgt ctttcagtag caagctgatt    5700
   gccaggggca ggcccttggt gtaagtgttt acaaagcggt taagctggga tgggtgcata    5760
   cgtggggata tgagatgcat cttggactgt atttttaggt tggctatgtt cccagccata    5820
   tccctccggg gattcatgtt gtgcagaacc accagcacag tgtatccggt gcacttggga    5880
   aatttgtcat gtagcttaga aggaaatgcg tggaagaact tggagacgcc cttgtgacct    5940
   ccaagatttt ccatgcattc gtccataatg atggcaatgg gcccacgggc ggcggcctgg    6000
   gcgaagatat ttctgggatc actaacgtca tagttgtgtt ccaggatgag atcgtcatag    6060
   gccattttta caaagcgcgg gcggagggtg ccagactgcg gtataatggt tccatccggc    6120
   ccaggggcgt agttaccctc acagatttgc atttcccacg ctttgagttc agatgggggg    6180
   atcatgtcta cctgcggggc gatgaagaaa acggtttccg ggtaggggga gatcagctgg    6240
   gaagaaagca ggttcctgag cagctgcgac ttaccgcagc cggtgggccc gtaaatcaca    6300
   cctattaccg ggtgcaactg gtagttaaga gagctgcagc tgccgtcatc cctgagcagg    6360
   ggggccactt cgttaagcat gtccctgact cgcatgtttt ccctgaccaa atccgccaga    6420
   aggcgctcgc cgcccagcga tagcagttct tgcaaggaag caaagttttt caacggtttg    6480
   agaccgtccg ccgtaggcat gcttttgagc gtttgaccaa gcagttccag gcggtcccac    6540
   agctcggtca cctgctctac ggcatctcga tccagcatat ctcctcgttt cgcgggttgg    6600
   ggcggctttc gctgtacggc agtagtcggt gctcgtccag acgggccagg gtcatgtctt    6660
   tccacgggcg cagggtcctc gtcagcgtag tctgggtcac ggtgaagggg tgcgctccgg    6720
   gctgcgcgct ggccagggtg cgcttgaggc tggtcctgct ggtgctgaag cgctgccggt    6780
   cttcgccctg cgcgtcggcc aggtagcatt tgaccatggt gtcatagtcc agcccctccg    6840
   cggcgtggcc cttggcgcgc agcttgccct tggaggaggc gccgcacgag gggcagtgca     6900
   gacttttgag ggcgtagagc ttgggcgcga gaaataccga ttccggggag taggcatccg    6960
   cgccgcaggc cccgcagacg gtctcgcatt ccacgagcca ggtgagctct ggccgttcgg    7020
   ggtcaaaaac caggtttccc ccatgctttt tgatgcgttt cttacctctg gtttccatga    7080
   gccggtgtcc acgctcggtg acgaaaaggc tgtccgtgtc cccgtataca gacttgagag    7140
   ggagtttaaa cgaattcaat agcttgttgc atgggcggcg atataaaatg caaggtgctg    7200
   ctcaaaaaat caggcaaagc ctcgcgcaaa aaagaaagca catcgtagtc atgctcatgc    7260
   agataaaggc aggtaagctc cggaaccacc acagaaaaag acaccatttt tctctcaaac    7320
   atgtctgcgg gtttctgcat aaacacaaaa taaaataaca aaaaaacatt taaacattag    7380
   aagcctgtct tacaacagga aaaacaaccc ttataagcat aagacggact acggccatgc    7440
   cggcgtgacc gtaaaaaaac tggtcaccgt gattaaaaag caccaccgac agctcctcgg    7500
   tcatgtccgg agtcataatg taagactcgg taaacacatc aggttgattc atcggtcagt    7560
   gctaaaaagc gaccgaaata gcccggggga atacataccc gcaggcgtag agacaacatt    7620
   acagccccca taggaggtat aacaaaatta ataggagaga aaaacacata aacacctgaa    7680
   aaaccctcct gcctaggcaa aatagcaccc tcccgctcca gaacaacata cagcgcttca    7740
   cagcggcagc ctaacagtca gccttaccag taaaaaagaa aacctattaa aaaaacacca    7800
   ctcgacacgg caccagctca atcagtcaca gtgtaaaaaa gggccaagtg cagagcgagt    7860
   atatatagga ctaaaaaatg acgtaacggt taaagtccac aaaaaacacc cagaaaaccg    7920
   cacgcgaacc tacgcccaga aacgaaagcc aaaaaaccca caacttcctc aaatcgtcac    7980
   ttccgttttc ccacgttacg taacttccca ttttaagaaa actacaattc ccaacacata    8040
   caagttactc cgccctaaaa cctacgtcac ccgccccgtt cccacgcccc gcgccacgtc    8100
   acaaactcca ccccctcatt atcatattgg cttcaatcca aaataaggta tattattgat    8160
   gatgttaatt aacatgcatg gatccatatg cggtgtgaaa taccgcacag atgcgtaagg    8220
   agaaaatacc gcatcaggcg ctcttccgct tcctcgctca ctgactcgct gcgctcggtc    8280
   gttcggctgc ggcgagcggt atcagctcac tcaaaggcgg taatacggtt atccacagaa    8340
   tcaggggata acgcaggaaa gaacatgtga gcaaaaggcc agcaaaaggc caggaaccgt    8400
   aaaaaggccg cgttgctggc gtttttccat aggctccgcc cccctgacga gcatcacaaa    8460
   aatcgacgct caagtcagag gtggcgaaac ccgacaggac tataaagata ccaggcgttt    8520
   ccccctggaa gctccctcgt gcgctctcct gttccgaccc tgccgcttac cggatacctg    8580
   tccgcctttc tcccttcggg aagcgtggcg ctttctcata gctcacgctg taggtatctc    8640
   agttcggtgt aggtcgttcg ctccaagctg ggctgtgtgc acgaaccccc cgttcagccc    8700
   gaccgctgcg ccttatccgg taactatcgt cttgagtcca acccggtaag acacgactta    8760
   tcgccactgg cagcagccac tggtaacagg attagcagag cgaggtatgt aggcggtgct    8820
   acagagttct tgaagtggtg gcctaactac ggctacacta gaaggacagt atttggtatc    8880
   tgcgctctgc tgaagccagt taccttcgga aaaagagttg gtagctcttg atccggcaaa    8940
   caaaccaccg ctggtagcgg tggttttttt gtttgcaagc agcagattac gcgcagaaaa    9000
   aaaggatctc aagaagatcc tttgatcttt tctacggggt ctgacgctca gtggaacgaa    9060
   aactcacgtt aagggatttt ggtcatgaga ttatcaaaaa ggatcttcac ctagatcctt    9120
   ttaaattaaa aatgaagttt taaatcaatc taaagtatat atgagtaaac ttggtctgac    9180
   agttaccaat gcttaatcag tgaggcacct atctcagcga tctgtctatt tcgttcatcc    9240
   atagttgcct gactccccgt cgtgtagata actacgatac gggagggctt accatctggc    9300
   cccagtgctg caatgatacc gcgagaccca cgctcaccgg ctccagattt atcagcaata    9360
   aaccagccag ccggaagggc cgagcgcaga agtggtcctg caactttatc cgcctccatc    9420
   cagtctatta attgttgccg ggaagctaga gtaagtagtt cgccagttaa tagtttgcgc    9480
   aacgttgttg ccattgctgc agccatgaga ttatcaaaaa ggatcttcac ctagatcctt    9540
   ttcacgtaga aagccagtcc gcagaaacgg tgctgacccc ggatgaatgt cagctactgg    9600
   gctatctgga caagggaaaa cgcaagcgca aagagaaagc aggtagcttg cagtgggctt    9660
   acatggcgat agctagactg ggcggtttta tggacagcaa gcgaaccgga attgccagct    9720
   ggggcgccct ctggtaaggt tgggaagccc tgcaaagtaa actggatggc tttctcgccg    9780
   ccaaggatct gatggcgcag gggatcaagc tctgatcaag agacaggatg aggatcgttt    9840
   cgcatgattg aacaagatgg attgcacgca ggttctccgg ccgcttgggt ggagaggcta    9900
   ttcggctatg actgggcaca acagacaatc ggctgctctg atgccgccgt gttccggctg   9960
   tcagcgcagg ggcgcccggt tctttttgtc aagaccgacc tgtccggtgc cctgaatgaa  10020
   ctgcaagacg aggcagcgcg gctatcgtgg ctggccacga cgggcgttcc ttgcgcagct  10080
   gtgctcgacg ttgtcactga agcgggaagg gactggctgc tattgggcga agtgccgggg  10140
   caggatctcc tgtcatctca ccttgctcct gccgagaaag tatccatcat ggctgatgca  10200
   atgcggcggc tgcatacgct tgatccggct acctgcccat tcgaccacca agcgaaacat  10260
   cgcatcgagc gagcacgtac tcggatggaa gccggtcttg tcgatcagga tgatctggac  10320
   gaagagcatc aggggctcgc gccagccgaa ctgttcgcca ggctcaaggc gagcatgccc  10380
   gacggcgagg atctcgtcgt gacccatggc gatgcctgct tgccgaatat catggtggaa  10440
   aatggccgct tttctggatt catcgactgt ggccggctgg gtgtggcgga ccgctatcag  10500
   gacatagcgt tggctacccg tgatattgct gaagagcttg gcggcgaatg ggctgaccgc  10560
   ttcctcgtgc tttacggtat cgccgctccc gattcgcagc gcatcgcctt ctatcgcctt  10620
   cttgacgagt tcttctgaat tttgttaaaa tttttgttaa atcagctcat ttttaaccca  10680
   ataggccgaa atcggcaaca tcccttataa atcaaaagaa tagaccgcga tagggttgag  10740
   tgttgttcca gtttggaaca agagtccact attaaagaac gtggactcca acgtcaaagg  10800
   gcgaaaaacc gtctatcagg gcgatggccc actacgtgaa ccatcaccca aatcaagttt  10860
   tttgcggtcg aggtgccgta aagctctaaa tcggaaccct aaagggagcc cccgatttag  10920
   agcttgacgg ggaaagccgg cgaacgtggc gagaaaggaa gggaagaaag cgaaaggagc  10980
   gggcgctagg gcgctggcaa gtgtagcggt cacgctgcgc gtaaccacca cacccgcgcg  11040
   cttaatgcgc cgctacaggg cgcgtccatt cgccattcag gatcgaatta attcttaagt  11100
   ttttbtaaca tcatcaataa tataccttat tttggattga agccaatatg ataatgaggg  11160
   ggtggagttt gtgacgtggc gcggggcgtg ggaacggggc gggtgacgta gtagtgtggc  11220
   ggaagtgtga tgttgcaagt gtggcggaac acatgtaagc gacggatgtg gcaaaagtga  11280
   cgtttttggt gtgcgccggt gtacacagga agtgacaatt ttcgcgcggt tttaggcgga  11340
   tgttgtagta aatttgggcg taaccgagta agatttggcc attttcgcgg gaaaactgaa  11400
   taagaggaag tgaaatctga ataattttgt gttactcata gcgcgtaata ctg         11453
<210> 4
<211> 9784
   <212> DNA
   <213> Artificial
<220>
   <223> Adenovirales Expressionsplasmid pAd.3r zur regulierten Expression
<220>
   <221> gene
   <222> (327)..(713)
   <223> VP16
<220>
   <221> gene
   <222> (714)..(1352)
   <223> TetR
<220>
   <221> Intron
   <222> (1353)..(1912)
   <223>
<220>
   <221> promoter
   <222> (1864)..(1902)
   <223> TK-min
<220>
   <221> protein_bind
   <222> (1913)..(2212)
   <223> TetO7
<220>
   <221> misc_feature
   <222> (2213)..(2709)
   <223> CMV-min + Intron
<220>
   <221> promoter
   <222> (2226)..(2264)
   <223> CMV-min
<220>
   <221> multiple cloning site
   <222> (2669)..(2770)
   <223> NheI, XhoI, KpnI, BamHI, SpeI, EcoRV, NotI, XhoI
<400> 4
   ctgctggttc tttccgcctc agaagccata gagcccaccg catccccagc atgcctgcta 60
   ttgtcttccc aatcctcccc cttgctgtcc tgccccaccc caccccccag aatagaatga 120
   cacctactca gacaatgcga tgcaatttcc tcattttatt aggaaaggac agtgggagtg 180
   gcaccttcca gggtcaagga aggcacgggg gaggggcaaa caacagatgg ctggcaacta 240
   gaaggcacag tcgaggctga tcagcgagct ctagcattta ggtgacacta tagaataggg 300
   ccctctagga tcgatcctcg cgccccctac ccaccgtact cgtcaattcc aagggcatcg 360
   gtaaacatct gctcaaactc gaagtcggcc atatccagag cgccgtaggg ggcggagtcg 420
   tggggggtaa atcccggacc cggggaatcc ccgtccccca acatgtccag atcgaaatcg 480
   tctagcgcgt cggcatgcgc catcgccacg tcctcgccgt ctaagtggag ctcgtccccc 540
   aggctgacat cggtcggggg ggccgtggac agtctgcgcg tgtgtcccgc ggggagaaag 600
   gacaggcgcg gagccgccag ccccgcctct tcgggggcgt cgtcgtccgg gagatcgagc 660
   aggccctcga tggtagaccc gtaattgttt ttcgtacgcg cgcggctgta cgcggaccca 720
   ctttcacatt taagttgttt ttctaatccg catatgatca attcaaggcc gaataagaag 780
   gctggctctg caccttggtg atcaaataat tcgatagctt gtcgtaataa tggcggcata 840
   ctatcagtag taggtgtttc cctttcttct ttagcgactt gatgctcttg atcttccaat 900
   acgcaaccta aagtaaaatg ccccacagcg ctgagtgcat ataatgcatt ctctagtgaa 960
   aaaccttgtt ggcataaaaa ggctaattga ttttcgagag tttcatactg tttttctgta 1020
   ggccgtgtac ctaaatgtac ttttgctcca tcgcgatgac ttagtaaagc acatctaaaa 1080
   cttttagcgt tattacgtaa aaaatcttgc cagctttccc cttctaaagg gcaaaagtga 1140
   gtatggtgcc tatctaacat ctcaatggct aaggcgtcga gcaaagcccg cttatttttt 1200
   acatgccaat acaatgtagg ctgctctaca cctagcttct gggcgagttt acgggttgtt 1260
   aaaccttcga ttccgacctc attaagcagc tctaatgcgc tgttaatcac tttactttta 1320
   tctaatctag agggtctggg tctctttggc atggtcgaat taattcgcgt cgagccggcc 1380
   gcgggtacaa ttccggttgg acctgggagt ggacacctgt ggagagaaag gcaaagtgga 1440
   tgtcattgtc actcaagtgt atggccagat ctcaagcctg ccacacctca agcttgacaa 1500
   caaaaagatt gtcttttctg accagatgga cgcggccacc ctcaaaggca tcaccgcggg 1560
   ccaggtgaat atcaaatcct cctcgttttt ggaaactgac aatcttagcg cagaagtcat 1620
   gcccgctttt gagagggagt actcacccca acagtcgaga ggttttccga tccggtcgat 1680
   gcggactcgc tcaggtccct cggtggcgga gtaccgttcg gaggccgacg ggtttccgat 1740
   ccaagagtac tggaaagacc gcgaagagtt tgtcctcaac cgcgagccca acaggcgtcg 1800
   aagcttgatg ggtcgctcgg tgttcgaggc cacacgcgtc accttaatat gcgaagtgga 1860
   cctcggaccg cgccgccccg actgcatctg cgtgttcgaa ttgcccggcg agctcgactt 1920
   tcacttttct ctatcactga tagggagtgg taaactcgac tttcactttt ctctatcact 1980
   gatagggagt ggtaaactcg actttcactt ttctctatca ctgataggga gtggtaaact 2040
   cgactttcac ttttctctat cactgatagg gagtggtaaa ctcgactttc acttttctct 2100
   atcactgata gggagtggta aactcgactt tcacttttct ctatcactga tagggagtgg 2160
   taaactcgac tttcactttt ctctatcact gatagggagt ggtaaactcg acggtcgagg 2220
   gtcgagtagg cgtgtacggt gggaggccta tataagcaga gctcgtttag tgaaccgtca 2280
   gatcgcctgg agacgccatc cacgctgttt tgacctccat agaagacacc gggaccgatc 2340
   cagcctccgc ggccccgaat tgcgaagctt tattgcggta gtttatcaca gttaaattgc 2400
   taacgcagtc agtgcttctg acacaacagt ctcgaactta agctgcagaa gttggtcgtg 2460
   aggcactggg caggtaagta tcaaggttac aagacaggtt taaggagacc aatagaaact 2520
   gggcttgtcg agacagagaa gactcttgcg tttctgatag gcacctattg gtcttactga 2580
   catccacttt gcctttctct ccacaggtgt ccactcccag ttcaattaca gctcttaagg 2640
   ctagagtact taatacgact cactataggc tagcctcgag aattcacgcg tggtaccgag 2700
   ctcggatcca ctagtaacgg ccgccagtgt gctggaattc tgcagatatc catcacactg 2760
   gcggccgctc gagcatgcat ctagagggcc ctattctata gtgtcaccta aatgctagag 2820
   ctcgctgatc agcctcgact gtgccttcta gttgccagcc atctgttgtt tgcccctccc 2880
   ccgtgccttc cttgaccctg gaaggtgcca ctcccactgt cctttcctaa taaaatgagg 2940
   aaattgcatc gcattgtctg agtaggtgtc attctattct ggggggtggg gtggggcagg 3000
   acagcaaggg ggaggattgg gaagacaata gcaggcatgc tggggatgcg gtgggctcta 3060
   tggcttctga ggcggaaaga accagtcgac atcgatgcta gagtggaagg tgctgaggta 3120
   cgatgagacc cgcaccaggt gcagaccctg cgagtgtggc ggtaaacata ttaggaacca 3180
   gcctgtgatg ctggatgtga ccgaggagct gaggcccgat cacttggtgc tggcctgcac 3240
   ccgcgctgag tttggctcta gcgatgaaga tacagattga ggtactgaaa tgtgtgggcg 3300
   tggcttaagg gtgggaaaga atatataagg tgggggtctt atgtagtttt gtatctgttt 3360
   tgcagcagcc gccgccgcca tgagcaccaa ctcgtttgat ggaagcattg tgagctcata 3420
   tttgacaacg cgcatgcccc catgggccgg ggtgcgtcag aatgtgatgg gctccagcat 3480
   tgatggtcgc cccgtcctgc ccgcaaactc tactaccttg acctacgaga ccgtgtctgg 3540
   aacgccgttg gagactgcag cctccgccgc cgcttcagcc gctgcagcca ccgcccgcgg 3600
   gattgtgact gactttgctt tcctgagccc gcttgcaagc agtgcagctt cccgttcatc 3660
   cgcccgcgat gacaagttga cggctctttt ggcacaattg gattctttga cccgggaact 3720
   taatgtcgtt tctcagcagc tgttggatct gcgccagcag gtttctgccc tgaaggcttc 3780
   ctcccctccc aatgcggttt aaaacataaa taaaaaacca gactctgttt ggatttggat 3840
   caagcaagtg tcttgctgtc tttatttagg ggttttgcgc gcgcggtagg cccgggacca 3900
   gcggtctcgg tcgttgaggg tcctgtgtat tttttccagg acgtggtaaa ggtgactctg 3960
   gatgttcaga tacatgggca taagcccgtc tctggggtgg aggtagcacc actgcagagc 4020
   ttcatgctgc ggggtggtgt tgtagatgat ccagtcgtag caggagcgct gggcgtggtg 4080
   cctaaaaatg tctttcagta gcaagctgat tgccaggggc aggcccttgg tgtaagtgtt 4140
   tacaaagcgg ttaagctggg atgggtgcat acgtggggat atgagatgca tcttggactg 4200
   tatttttagg ttggctatgt tcccagccat atccctccgg ggattcatgt tgtgcagaac 4260
   caccagcaca gtgtatccgg tgcacttggg aaatttgtca tgtagcttag aaggaaatgc 4320
   gtggaagaac ttggagacgc ccttgtgacc tccaagattt tccatgcatt cgtccataat 4380
   gatggcaatg ggcccacggg cggcggcctg ggcgaagata tttctgggat cactaacgtc 4440
   atagttgtgt tccaggatga gatcgtcata ggccattttt acaaagcgcg ggcggagggt 4500
   gccagactgc ggtataatgg ttccatccgg cccaggggcg tagttaccct cacagatttg 4560
   catttcccac gctttgagtt cagatggggg gatcatgtct acctgcgggg cgatgaagaa 4620
   aacggtttcc ggggtagggg agatcagctg ggaagaaagc aggttcctga gcagctgcga 4680
   cttaccgcag ccggtgggcc cgtaaatcac acctattacc gggtgcaact ggtagttaag 4740
   agagctgcag ctgccgtcat ccctgagcag gggggccact tcgttaagca tgtccctgac 4800
   tcgcatgttt tccctgacca aatccgccag aaggcgctcg ccgcccagcg atagcagttc 4860
   ttgcaaggaa gcaaagtttt tcaacggttt gagaccgtcc gccgtaggca tgcttttgag 4920
   cgtttgacca agcagttcca ggcggtccca cagctcggtc acctgctcta cggcatctcg 4980
   atccagcata tctcctcgtt tcgcgggttg ggcggctttc cgctgtacgg cagtagtcgg 5040
   tgctcgtcca gacgggccag ggtcatgtct ttccacgggc gcagggtcct cgtcagcgta 5100
   gtctgggtca cggtgaaggg gtgcgctccg ggctgcgcgc tggccagggt gcgcttgagg 5160
   ctggtcctgc tggtgctgaa gcgctgccgg tcttcgccct gcgcgtcggc caggtagcat 5220
   ttgaccatgg tgtcatagtc cagcccctcc gcggcgtggc ccttggcgcg cagcttgccc 5280
   ttggaggagg cgccgcacga ggggcagtgc agacttttga gggcgtagag cttgggcgcg 5340
   agaaataccg attccgggga gtaggcatcc gcgccgcagg ccccgcagac ggtctcgcat 5400
   tccacgagcc aggtgagctc tggccgttcg gggtcaaaaa ccaggtttcc cccatgcttt 5460
   ttgatgcgtt tcttacctct ggtttccatg agccggtgtc cacgctcggt gacgaaaagg 5520
   ctgtccgtgt ccccgtatac agacttgaga ggcctgtcct cgaccgatgc ccttgagagc 5580
   cttcaaccca gtcagctcct tccggtgggc gcggggcatg actatcgtcg ccgcacttat 5640
   gactgtcttc tttatcatgc aactcgtagg acaggtgccg gcagcgctct gggtcatttt 5700
   cggcgaggac cgctttcgct ggagcgcgac gatgatcggc ctgtcgcttg cggtattcgg 5760
   aatcttgcac gccctcgctc aagccttcgt cactggtccc gccaccaaac gtttcggcga 5820
   gaagcaggcc attatcgccg gcatggcggc cgacgcgctg ggctacgtct tgctggcgtt 5880
   cgcgacgcga ggctggatgg ccttccccat tatgattctt ctcgcttccg gcggcatcgg 5940
   gatgcccgcg ttgcaggcca tgctgtccag gcaggtagat gacgaccatc agggacagct 6000
   tcaaggatcg ctcgcggctc ttaccagcct aacttcgatc actggaccgc tgatcgtcac 6060
   ggcgatttat gccgcctcgg cgagcacatg gaacgggttg gcatggattg taggcgccgc 6120
   cctatacctt gtctgcctcc ccgcgttgcg tcgcggtgca tggagccggg ccacctcgac 6180
   ctgaatggaa gccggcggca cctcgctaac ggattcacca ctccaagaat tggagccaat 6240
   caattcttgc ggagaactgt gaatgcgcaa accaaccctt ggcagaacat atccatcgcg  6300
   tccgccatct ccagcagccg cacgcggcgc atctcgggca gcgttgggtc ctggccacgg  6360
   gtgcgcatga tcgtgctcct gtcgttgagg acccggctag gctggcgggg ttgccttact  6420
   ggttagcaga atgaatcacc gatacgcgag cgaacgtgaa gcgactgctg ctgcaaaacg  6480
   tctgcgacct gagcaacaac atgaatggtc ttcggtttcc gtgtttcgta aagtctggaa  6540
   acgcggaagt cagcgccctg caccattatg ttccggatct gcatcgcagg atgctgctgg  6600
   ctaccctgtg gaacacctac atctgtatta acgaagcgct ggcattgacc ctgagtgatt  6660
   tttctctggt cccgccgcat ccataccgcc agttgtttac cctcacaacg ttccagtaac  6720
   cgggcatgtt catcatcagt aacccgtatc gtgagcatcc tctctcgttt catcggtatc  6780
   attaccccca tgaacagaaa ttccccctta cacggaggca tcaagtgacc aaacaggaaa  6840
   aaaccgccct taacatggcc cgctttatca gaagccagac attaacgctt ctggagaaac  6900
   tcaacgagct ggacgcggat gaacaggcag acatctgtga atcgcttcac gaccacgctg  6960
   atgagcttta ccgcagctgc ctcgcgcgtt tcggtgatga cggtgaaaac ctctgacaca  7020
   tgcagctccc ggagacggtc acagcttgtc tgtaagcgga tgccgggagc agacaagccc  7080
   gtcagggcgc gtcagcgggt gttggcgggt gtcggggcgc agccatgacc cagtcacgta  7140
   gcgatagcgg agtgtatact ggcttaacta tgcggcatca gagcagattg tactgagagt  7200
   gcaccatatg cggtgtgaaa taccgcacag atgcgtaagg agaaaatacc gcatcaggcg  7260
   ctcttccgct tcctcgctca ctgactcgct gcgctcggtc gttcggctgc ggcgagcggt  7320
   atcagctcac tcaaaggcgg taatacggtt atccacagaa tcaggggata acgcaggaaa  7380
   gaacatgtga gcaaaaggcc agcaaaaggc caggaaccgt aaaaaggccg cgttgctggc  7440
   gtttttccat aggctccgcc cccctgacga gcatcacaaa aatcgacgct caagtcagag  7500
   gtggcgaaac ccgacaggac tataaagata ccaggcgttt ccccctggaa gctccctcgt  7560
   gcgctctcct gttccgaccc tgccgcttac cggatacctg tccgcctttc tcccttcggg  7620
   aagcgtggcg ctttctcaat gctcacgctg taggtatctc agttcggtgt aggtcgttcg  7680
   ctccaagctg ggctgtgtgc acgaaccccc cgttcagccc gaccgctgcg ccttatccgg  7740
   taactatcgt cttgagtcca acccggtaag acacgactta tcgccactgg cagcagccac  7800
   tggtaacagg attagcagag cgaggtatgt aggcggtgct acagagttct tgaagtggtg  7860
   gcctaactac ggctacacta gaaggacagt atttggtatc tgcgctctgc tgaagccagt  7920
   taccttcgga aaaagagttg gtagctcttg atccggcaaa caaaccaccg ctggtagcgg  7980
   tggttttttt gtttgcaagc agcagattac gcgcagaaaa aaaggatctc aagaagatcc  8040
   tttgatcttt tctacggggt ctgacgctca gtggaacgaa aactcacgtt aagggatttt  8100
   ggtcatgaga ttatcaaaaa ggatcttcac ctagatcctt ttaaattaaa aatgaagttt  8160
   taaatcaatc taaagtatat atgagtaaac ttggtctgac agttaccaat gcttaatcag  8220
   tgaggcacct atctcagcga tctgtctatt tcgttcatcc atagttgcct gactccccgt  8280
   cgtgtagata actacgatac gggagggctt accatctggc cccagtgctg caatgatacc  8340
   gcgagaccca cgctcaccgg ctccagattt atcagcaata aaccagccag ccggaagggc  8400
   cgagcgcaga agtggtcctg caactttatc cgcctccatc cagtctatta attgttgccg  8460
   ggaagctaga gtaagtagtt cgccagttaa tagtttgcgc aacgttgttg ccattgctgc  8520
   aggcatcgtg gtgtcacgct cgtcgtttgg tatggcttca ttcagctccg gttcccaacg  8580
   atcaaggcga gttacatgat cccccatgtt gtgcaaaaaa gcggttagct ccttcggtcc  8640
   tccgatcgtt gtcagaagta agttggccgc agtgttatca ctcatggtta tggcagcact  8700
   gcataattct cttactgtca tgccatccgt aagatgcttt tctgtgactg gtgagtactc  8760
   aaccaagtca ttctgagaat agtgtatgcg gcgaccgagt tgctcttgcc cggcgtcaac  8820
   acgggataat accgcgccac atagcagaac tttaaaagtg ctcatcattg gaaaacgttc  8880
   ttcggggcga aaactctcaa ggatcttacc gctgttgaga tccagttcga tgtaacccac  8940
   tcgtgcaccc aactgatctt cagcatcttt tactttcacc agcgtttctg ggtgagcaaa  9000
   aacaggaagg caaaatgccg caaaaaaggg aataagggcg acacggaaat gttgaatact  9060
   catactcttc ctttttcaat attattgaag catttatcag ggttattgtc tcatgagcgg  9120
   atacatattt gaatgtattt agaaaaataa acaaataggg gttccgcgca catttccccg  9180
   aaaagtgcca cctgacgtct aagaaaccat tattatcatg acattaacct ataaaaatag  9240
   gcgtatcacg aggccctttc gtcttcaaga attcttatca tgacattaac ctataaaaat  9300
   aggcgtatca cgaggccctt tcgtcatcat caataatata ccttattttg gattgaagcc  9360
   aatatgataa tgagggggtg gagtttgtga cgtggcgcgg ggcgtgggaa cggggcgggt  9420
   gacgtagtag tgtggcggaa gtgtgatgtt gcaagtgtgg cggaacacat gtaagcgccg  9480
   gatgtggtaa aagtgacgtt tttggtgtgc gccggtgtat acgggaagtg acaattttcg  9540
   cgcggtttta ggcggatgtt gtagtaaatt tgggcgtaac caagtaatgt ttggccattt  9600
   tcgcgggaaa actgaataag aggaagtgaa atctgaataa ttctgtgtta ctcatagcgc  9660
   gtaatatttg tctagggccg cggggacttt gaccgtttac gtggagactc gcccaggtgt  9720
   ttttctcagg tgttttccgc gttccgggtc aaagttggcg ttttattatt atagtcagct  9780
   ctag                                                               9784
<210> 5
   <211> 9668
   <212> DNA
   <213> Artificial
<220>
   <223> Expressionsplasmid pShuttle.3r zur regulierten Expression nach Virusgeneration mittels AdEasy
<220>
   <221> gene
   <222> (327)..(713)
   <223> VP16
<220>
   <221> gene
   <222> (714)..(1352)
   <223> TetR
<220>
   <221> Intron
   <222> (1353)..(1912)
   <223>
<220>
   <221> promoter
   <222> (1864)..(1902)
   <223> TK-min
<220>
   <221> protein_bind
   <222> (1913) .. (2212)
   <223> TetO7
<220>
   <221> misc_feature
   <222> (2213)..(2709)
   <223> CMV-min + Intron
<220>
   <221> promoter
   <222> (2226)..(2264)
   <223> CMV-min
<220>
   <221> multiple cloning site
   <222> (2669)..(2770)
   <223> NheI, XhoI, KpnI, SpeI, EcoRV, NotI, XhoI
<400> 5
   ctgctggttc tttccgcctc agaagccata gagcccaccg catccccagc atgcctgcta    60
   ttgtcttccc aatcctcccc cttgctgtcc tgccccaccc caccccccag aatagaatga    120
   cacctactca gacaatgcga tgcaatttcc tcattttatt aggaaaggac agtgggagtg    180
   gcaccttcca gggtcaagga aggcacgggg gaggggcaaa caacagatgg ctggcaacta    240
   gaaggcacag tcgaggctga tcagcgagct ctagcattta ggtgacacta tagaataggg    300
   ccctctagga tcgatcctcg cgccccctac ccaccgtact cgtcaattcc aagggcatcg    360
   gtaaacatct gctcaaactc gaagtcggcc atatccagag cgccgtaggg ggcggagtcg    420
   tggggggtaa atcccggacc cggggaatcc ccgtccccca acatgtccag atcgaaatcg    480
   tctagcgcgt cggcatgcgc catcgccacg tcctcgccgt ctaagtggag ctcgtccccc    540
   aggctgacat cggtcggggg ggccgtcgat agtctgcgcg tgtgtcccgc ggggagaaag    600
   gacaggcgcg gagccgccag ccccgcctct tcgggggcgt cgtcgtccgg gagatcgagc 660
   aggccctcga tggtagaccc gtaattgttt ttcgtacgcg cgcggctgta cgcggaccca 720
   ctttcacatt taagttgttt ttctaatccg catatgatca attcaaggcc gaataagaag 780
   gctggctctg caccttggtg atcaaataat tcgatagctt gtcgtaataa tggcggcata 840
   ctatcagtag taggtgtttc cctttcttct ttagcgactt gatgctcttg atcttccaat 900
   acgcaaccta aagtaaaatg ccccacagcg ctgagtgcat ataacgcgtt ctctagtgaa 960
   aaaccttgtt ggcataaaaa ggctaattga ttttcgagag tttcatactg tttttctgta 1020
   ggccgtgtat ctgaatgtac ttttgctcca ttgcgatgac ttagtaaagc acatctaaaa 1080
   cttttagcgt tattgcgtaa aaaatcttgc cagctttccc cttttaaagg gcaaaagtga 1140
   gtatggtgcc tatctaacat ctcaatggct aaggcgtcga gcaaagcccg cttatttttt 1200
   acatgccaat acagtgtagg ctgctctaca ccaagcttct gggcgagttt acgggttgtt 1260
   aaaccttcga ttccgacctc attaagcagc tctaatgcgc tgttaatcac tttactttta 1320
   tctaatctag agggtctggg tctctttggc atggtcgaat taattcgcgt cgagccggcc 1380
   gcgggtacaa ttccggttgg acctgggagt ggacacctgt ggagagaaag gcaaagtgga 1440
   tgtcattgtc actcaagtgt atggccagat ctcaagcctg ccacacctca agcttgacaa 1500
   caaaaagatt gtcttttctg accagatgga cgcggccacc ctcaaaggca tcaccgcggg 1560
   ccaggtgaat atcaaatcct cctcgttttt ggaaactgac aatcttagcg cagaagtcat 1620
   gcccgctttt gagagggagt actcacccca acagtcgaga ggttttccga tccggtcgat 1680
   gcggactcgc tcaggtccct cggtggcgga gtaccgttcg gaggccgacg ggtttccgat 1740
   ccaagagtac tggaaagacc gcgaagagtt tgtcctcaac cgcgagccca acaggcgtcg 1800
   aagcttgatg ggtcgctcgg tgttcgaggc cacacgcgtc accttaatat gcgaagtgga 1860
   cctcggaccg cgccgccccg actgcatctg cgtgttcgaa ttgcccggcg agctcgactt 1920
   tcacttttct ctatcactga tagggagtgg taaactcgac tttcactttt ctctatcact 1980
   gatagggagt ggtaaactcg actttcactt ttctctatca ctgataggga gtggtaaact 2040
   cgactttcac ttttctctat cactgatagg gagtggtaaa ctcgactttc acttttctct 2100
   atcactgata gggagtggta aactcgactt tcacttttct ctatcactga tagggagtgg 2160
   taaactcgac tttcactttt ctctatcact gatagggagt ggtaaactcg acggtcgagg 2220
   gtcgagtagg cgtgtacggt gggaggccta tataagcaga gctcgtttag tgaaccgtca 2280
   gatcgcctgg agacgccatc cacgctgttt tgacctccat agaagacacc gggaccgatc 2340
   cagcctccgc ggccccgaat tgcgaagctt tattgcggta gtttatcaca gttaaattgc 2400
   taacgcagtc agtgcttctg acacaacagt ctcgaactta agctgcagaa gttggtcgtg 2460
   aggcactggg caggtaagta tcaaggttac aagacaggtt taaggagacc aatagaaact 2520
   gggcttgtcg agacagagaa gactcttgcg tttctgatag gcacctattg gtcttactga 2580
   catccacttt gcctttctct ccacaggtgt ccactcccag ttcaattaca gctcttaagg 2640
   ctagagtact taatacgact cactataggc tagcctcgag aattcacgcg tggtaccgag 2700
   ctcggatcca ctagtaacgg ccgccagtgt gctggaattc tgcagatatc catcacactg 2760
   gcggccgctc gagcatgcat ctagagggcc ctattctata gtgtcaccta aatgctagag 2820
   ctcgctgatc agcctcgact gtgccttcta gttgccagcc atctgttgtt tgcccctccc 2880
   ccgtgccttc cttgaccctg gaaggtgcca ctcccactgt cctttcctaa taaaatgagg 2940
   aaattgcatc gcattgtctg agtaggtgtc attctattct ggggggtggg gtggggcagg 3000
   acagcaaggg ggaggattgg gaagacaata gcaggcatgc tggggatgcg gtgggctcta 3060
   tggcttctga ggcggaaaga accagtcgac tcgaagatct gggcgtggtt aagggtggga 3120
   aagaatatat aaggtggggg tcttatgtag ttttgtatct gttttgcagc agccgccgcc 3180
   gccatgagca ccaactcgtt tgatggaagc attgtgagct catatttgac aacgcgcatg 3240
   cccccatggg ccggggtgcg tcagaatgtg atgggctcca gcattgatgg tcgccccgtc 3300
   ctgcccgcaa actctactac cttgacctac gagaccgtgt ctggaacgcc gttggagact 3360
   gcagcctccg ccgccgcttc agccgctgca gccaccgccc gcgggattgt gactgacttt 3420
   gctttcctga gcccgcttgc aagcagtgca gcttcccgtt catccgcccg cgatgacaag 3480
   ttgacggctc ttttggcaca attggattct ttgacccggg aacttaatgt cgtttctcag 3540
   cagctgttgg atctgcgcca gcaggtttct gccctgaagg cttcctcccc tcccaatgcg 3600
   gtttaaaaca taaataaaaa accagactct gtttggattt ggatcaagca agtgtcttgc 3660
   tgtctttatt taggggtttt gcgcgcgcgg taggcccggg accagcggtc tcggtcgttg 3720
   agggtcctgt gtattttttc caggacgtgg taaaggtgac tctggatgtt cagatacatg 3780
   ggcataagcc cgtctctggg gtggaggtag caccactgca gagcttcatg ctgcggggtg 3840
   gtgttgtaga tgatccagtc gtagcaggag cgctgggcgt ggtgcctaaa aatgtctttc 3900
   agtagcaagc tgattgccag gggcaggccc ttggtgtaag tgtttacaaa gcggttaagc 3960
   tgggatgggt gcatacgtgg ggatatgaga tgcatcttgg actgtatttt taggttggct 4020
   atgttcccag ccatatccct ccggggattc atgttgtgca gaaccaccag cacagtgtat 4080
   ccggtgcact tgggaaattt gtcatgtagc ttagaaggaa atgcgtggaa gaacttggag 4140
   acgcccttgt gacctccaag attttccatg cattcgtcca taatgatggc aatgggccca 4200
   cgggcggcgg cctgggcgaa gatatttctg ggatcactaa cgtcatagtt gtgttccagg 4260
   atgagatcgt cataggccat ttttacaaag cgcgggcgga gggtgccaga ctgcggtata 4320
   atggttccat ccggcccagg ggcgtagtta ccctcacaga tttgcatttc ccacgctttg 4380
   agttcagatg gggggatcat gtctacctgc ggggcgatga agaaaacggt ttccggggta 4440
   ggggagatca gctgggaaga aagcaggttc ctgagcagct gcgacttacc gcagccggtg 4500
   ggcccgtaaa tcacacctat taccgggtgc aactggtagt taagagagct gcagctgccg 4560
   tcatccctga gcaggggggc cacttcgtta agcatgtccc tgactcgcat gttttccctg 4620
   accaaatccg ccagaaggcg ctcgccgccc agcgatagca gttcttgcaa ggaagcaaag 4680
   tttttcaacg gtttgagacc gtccgccgta ggcatgcttt tgagcgtttg accaagcagt 4740
   tccaggcggt cccacagctc ggtcacctgc tctacggcat ctcgatccag catatctcct 4800
   cgtttcgcgg gttggggcgg ctttcgctgt acggcagtag tcggtgctcg tccagacggg 4860
   ccagggtcat gtctttccac gggcgcaggg tcctcgtcag cgtagtctgg gtcacggtga 4920
   aggggtgcgc tccgggctgc gcgctggcca gggtgcgctt gaggctggtc ctgctggtgc 4980
   tgaagcgctg ccggtcttcg ccctgcgcgt cggccaggta gcatttgacc atggtgtcat 5040
   agtccagccc ctccgcggcg tggcccttgg cgcgcagctt gcccttggag gaggcgccgc 5100
   acgaggggca gtgcagactt ttgagggcgt agagcttggg cgcgagaaat accgattccg 5160
   gggagtaggc atccgcgccg caggccccgc agacggtctc gcattccacg agccaggtga 5220
   gctctggccg ttcggggtca aaaaccaggt ttcccccatg ctttttgatg cgtttcttac 5280
   ctctggtttc catgagccgg tgtccacgct cggtgacgaa aaggctgtcc gtgtccccgt 5340
   atacagactt gagagggagt ttaaacgaat tcaatagctt gttgcatggg cggcgatata 5400
   aaatgcaagg tgctgctcaa aaaatcaggc aaagcctcgc gcaaaaaaga aagcacatcg 5460
   tagtcatgct catgcagata aaggcaggta agctccggaa ccaccacaga aaaagacacc 5520
   atttttctct caaacatgtc tgcgggtttc tgcataaaca caaaataaaa taacaaaaaa 5580
   acatttaaac attagaagcc tgtcttacaa caggaaaaac aacccttata agcataagac 5640
   ggactacggc catgccggcg tgaccgtaaa aaaactggtc accgtgatta aaaagcacca 5700
   ccgacagctc ctcggtcatg tccggagtca taatgtaaga ctcggtaaac acatcaggtt 5760
   gattcatcgg tcagtgctaa aaagcgaccg aaatagcccg ggggaataca tacccgcagg 5820
   cgtagagaca acattacagc ccccatagga ggtataacaa aattaatagg agagaaaaac 5880
   acataaacac ctgaaaaacc ctcctgccta ggcaaaatag caccctcccg ctccagaaca 5940
   acatacagcg cttcacagcg gcagcctaac agtcagcctt accagtaaaa aagaaaacct 6000
   attaaaaaaa caccactcga cacggcacca gctcaatcag tcacagtgta aaaaagggcc 6060
   aagtgcagag cgagtatata taggactaaa aaatgacgta acggttaaag tccacaaaaa 6120
   acacccagaa aaccgcacgc gaacctacgc ccagaaacga aagccaaaaa acccacaact 6180
   tcctcaaatc gtcacttccg ttttcccacg ttacgtaact tcccatttta agaaaactac 6240
   aattcccaac acatacaagt tactccgccc taaaacctac gtcacccgcc ccgttcccac 6300
   gccccgcgcc acgtcacaaa ctccaccccc tcattatcat attggcttca atccaaaata 6360
   aggtatatta ttgatgatgt taattaacat gcatggatcc atatgcggtg tgaaataccg 6420
   cacagatgcg taaggagaaa ataccgcatc aggcgctctt ccgcttcctc gctcactgac 6480
   tcgctgcgct cggtcgttcg gctgcggcga gcggtatcag ctcactcaaa ggcggtaata 6540
   cggttatcca cagaatcagg ggataacgca ggaaagaaca tgtgagcaaa aggccagcaa 6600
   aaggccagga accgtaaaaa ggccgcgttg ctggcgtttt tccataggct ccgcccccct 6660
   gacgagcatc acaaaaatcg acgctcaagt cagaggtggc gaaacccgac aggactataa 6720
   agataccagg cgtttccccc tggaagctcc ctcgtgcgct ctcctgttcc gaccctgccg 6780
   cttaccggat acctgtccgc ctttctccct tcgggaagcg tggcgctttc tcatagctca 6840
   cgctgtaggt atctcagttc ggtgtaggtc gttcgctcca agctgggctg tgtgcacgaa 6900
   ccccccgttc agcccgaccg ctgcgcctta tccggtaact atcgtcttga gtccaacccg 6960
   gtaagacacg acttatcgcc actggcagca gccactggta acaggattag cagagcgagg 7020
   tatgtaggcg gtgctacaga gttcttgaag tggtggccta actacggcta cactagaagg 7080
   acagtatttg gtatctgcgc tctgctgaag ccagttacct tcggaaaaag agttggtagc 7140
   tcttgatccg gcaaacaaac caccgctggt agcggtggtt tttttgtttg caagcagcag 7200
   attacgcgca gaaaaaaagg atctcaagaa gatcctttga tcttttctac ggggtctgac 7260
   gctcagtgga acgaaaactc acgttaaggg attttggtca tgagattatc aaaaaggatc 7320
   ttcacctaga tccttttaaa ttaaaaatga agttttaaat caatctaaag tatatatgag 7380
   taaacttggt ctgacagtta ccaatgctta atcagtgagg cacctatctc agcgatctgt 7440
   ctatttcgtt catccatagt tgcctgactc cccgtcgtgt agataactac gatacgggag 7500
   ggcttaccat ctggccccag tgctgcaatg ataccgcgag acccacgctc accggctcca 7560
   gatttatcag caataaacca gccagccgga agggccgagc gcagaagtgg tcctgcaact 7620
   ttatccgcct ccatccagtc tattaattgt tgccgggaag ctagagtaag tagttcgcca 7680
   gttaatagtt tgcgcaacgt tgttgccatt gctgcagcca tgagattatc aaaaaggatc 7740
   ttcacctaga tccttttcac gtagaaagcc agtccgcaga aacggtgctg accccggatg 7800
   aatgtcagct actgggctat ctggacaagg gaaaacgcaa gcgcaaagag aaagcaggta 7860
   gcttgcagtg ggcttacatg gcgatagcta gactgggcgg ttttatggac agcaagcgaa 7920
   ccggaattgc cagctggggc gccctctggt aaggttggga agccctgcaa agtaaactgg   7980
   atggctttct cgccgccaag gatctgatgg cgcaggggat caagctctga tcaagagaca   8040
   ggatgaggat cgtttcgcat gattgaacaa gatggattgc acgcaggttc tccggccgct   8100
   tgggtggaga ggctattcgg ctatgactgg gcacaacaga caatcggctg ctctgatgcc   8160
   gccgtgttcc ggctgtcagc gcaggggcgc ccggttcttt ttgtcaagac cgacctgtcc   8220
   ggtgccctga atgaactgca agacgaggca gcgcggctat cgtggctggc cacgacgggc   8280
   gttccttgcg cagctgtgct cgacgttgtc actgaagcgg gaagggactg gctgctattg   8340
   ggcgaagtgc cggggcagga tctcctgtca tctcaccttg ctcctgccga gaaagtatcc   8400
   atcatggctg atgcaatgcg gcggctgcat acgcttgatc cggctacctg cccattcgac   8460
   caccaagcga aacatcgcat cgagcgagca cgtactcgga tggaagccgg tcttgtcgat   8520
   caggatgatc tggacgaaga gcatcagggg ctcgcgccag ccgaactgtt cgccaggctc   8580
   aaggcgagca tgcccgacgg cgaggatctc gtcgtgaccc atggcgatgc ctgcttgccg   8640
   aatatcatgg tggaaaatgg ccgcttttct ggattcatcg actgtggccg gctgggtgtg   8700
   gcggaccgct atcaggacat agcgttggct acccgtgata ttgctgaaga gcttggcggc   8760
   gaatgggctg accgcttcct cgtgctttac ggtatcgccg ctcccgattc gcagcgcatc   8820
   gccttctatc gccttcttga cgagttcttc tgaattttgt taaaattttt gttaaatcag   8880
   ctcatttttt aaccaatagg ccgaaatcgg caacatccct tataaatcaa aagaatagac   8940
   cgcgataggg ttgagtgttg ttccagtttg gaacaagagt ccactattaa agaacgtgga   9000
   ctccaacgtc aaagggcgaa aaaccgtcta tcagggcgat ggcccactac gtgaaccatc   9060
   acccaaatca agttttttgc ggtcgaggtg ccgtaaagct ctaaatcgga accctaaagg   9120
   gagcccccga tttagagctt gacggggaaa gccggcgaac gtggcgagaa aggaagggaa   9180
   gaaagcgaaa ggagcgggcg ctagggcgct ggcaagtgta gcggtcacgc tgcgcgtaac   9240
   caccacaccc gcgcgcttaa tgcgccgcta cagggcgcgt ccattcgcca ttcaggatcg   9300
   aattaattct taagtttttb taacatcatc aataatatac cttattttgg attgaagcca   9360
   atatgataat gagggggtgg agtttgtgac gtggcgcggg gcgtgggaac ggggcgggtg   9420
   acgtagtagt gtggcggaag tgtgatgttg caagtgtggc ggaacacatg taagcgacgg   9480
   atgtggcaaa agtgacgttt ttggtgtgcg ccggtgtaca caggaagtga caattttcgc   9540
   gcggttttag gcggatgttg tagtaaattt gggcgtaacc gagtaagatt tggccatttt   9600
   cgcgggaaaa ctgaataaga ggaagtgaaa tctgaataat tttgtgttac tcatagcgcg   9660
   taatactg                                                            9668

## Beschreibung

Die Erfindung betrifft rekombinante virale Vektoren, die durch Tetracyclin oder Tetracyclin-Derivate, wie z.B. Doxycyclin, hocheffizient supprimiert werden können, sowie deren Verwendung zur Durchführung einer Genexpression in Eukaryontenzellen, insbesondere im Rahmen einer Gentherapie.

Bösartige Erkrankungen sind eine der häufigsten Todesursachen des Menschen. Bei fortgeschrittenen und metastasierten soliden Tumorerkrankungen sind die therapeutischen Möglichkeiten immer noch sehr limitiert und das 5-Jahresüberleben vieler dieser Karzinomerkrankungen beträgt weniger als 10%. Daher stellt die metastasierte Karzinomerkrankung eine der größten Herausforderungen in der experimentellen Medizin dar. Durch Einschleusen therapeutischer Gene in Tumorzellen eröffneten gentherapeutische Ansätze neue Perspektiven in der Therapie dieser Erkrankungen.

Adenoviren ermöglichen den effizienten Transfer und die Expression therapeutischer Gene in verschiedene Gewebe und Zelllinien. Insbesondere die Weiterentwicklung rekombinanter adenoviraler Vektoren hat die experimentellen Ansätze in der adenoviralen Gentherapie maligner Erkrankungen ermöglicht (K. Kozarsky, Curr Opin Genet Dev 3 (1993) 499-503).

Mit der hohen Effizienz des Gentransfers sind gentherapeutische Ansätze heute häufig durch die Toxizität infolge unkontrollierter Transgenexpression limitiert. Insbesondere bei der adenoviral vermittelten Expression von Zytokinen wie Interleukin-2, Interleukin-12, Interleukin-18 oder Tumor Nekrose Faktor α kann es auch bei intratumoraler Gabe der rekombinanten Adenoviren zu unerwarteten erheblichen systemischen Nebenwirkungen kommen. Eine den konstitutiven Promotoren (Cytomegalovirus-Promotor) vergleichbare Genexpression nach erfolgter adenoviraler Infektion mit rekombinanten Vektoren konnte bislang nicht effizient kontrolliert werden.

Gegenwärtig stellt das von M. Gossen et al. entwickelte Tet-System eines der am meisten geeigneten Mittel zur Kontrolle der Genexpression dar (M. Gossen et al., PNAS USA 89 (1992) 5547-5551; M. Gossen et al. Science 268 (1995) 1766-1769). Das Tet-System basiert auf zwei Elementen des E. coli Tet-Operons. Das Tetracyclin-induzierbare Repressorprotein (tetR) wird mit der transkriptionellen Aktivierungsdomäne des Herpes simplex Virus VP16 fusioniert. Dieses tTA Fusionsprotein interagiert mit der heptamerisierten tetO Operatorsequenz, was in der transkriptionellen Aktivierung der flankierenden minimalen Promotoren resultiert. Die Bindung von Tetracyclin und dessen Derivaten an die TetR-Domäne von tTA inhibiert die Wechselwirkung des Fusionsproteins mit dessen Operatorsequenzen, was zur Herunterregulierung der Transgen-Expression führt.

Die Verwendung des ursprünglichen tet-regulierten Genexpressionssystems in rekombinanten adenoviralen Vektoren (TC Harding et al. J. Neurochem. 69 (1997) 2620-2623; TC Harding et al. Nat. Biotechnol. 16 (1998) 553-555) brachte zwei wesentliche Hindernisse mit sich. Die limitierte Verpackungskapazität resultierte in unzureichender Aktivierung des CMV minimalen Promotors durch Verstärkungsinterferenz (S. Rubinchic et al. Gene Therapy 8 (2000) 875-885), und die konstitutionelle Transaktivator-Expression hatte eine mit VP16 zusammenhängende Toxizität zur Folge.

Von Nakagawa *et al*. (European Journal of Pharmaceutical Sciences 13 (2001) 53-60) wurde ein Zwei-Vektor-System offenbart, bei dem der eine Vektor ein Konstrukt zur konstitutiren Expression des tTA, also die CMV-vermittellte Expression eines TetR- VP16 Fusionsproteins, beinhaltet. Der andere Vektor beinhaltet das Tet-responsive Element (TetO₇), das von einem Minimalpromotor frankiert ist und der Expression eines therapeutischen Gens (IL-12) client.

Aufgabe der vorliegenden Erfindung ist es daher, ein für die Gentherapie von Tumorerkrankungen geeignetes Genexpressionssystem bereitzustellen, das die aus dem Stand der Technik bekannten Nachteile nicht aufweist. Insbesondere sollen (adeno-)virale Vektoren mit einem hohen Maß an Transgen-Expression bereitgestellt werden, die ferner die Möglichkeit bieten, dass diese Expression im Fall schwerwiegender Nebenwirkungen, die aus der Transgenexpression resultieren, effizient herunterreguliert werden kann. Vor allem sollen die Supprimierbarker 7 und Genexpression, die bei dem Zwei-Vektor-System von Nakagawd *et al*. (loc. cit.) beabachtet werden, verbessert werden. Ferner sollen die Vektoren ein hohes Maß an Sicherheit bei der Anwendung aufweisen, insbesondere soll die aus dem Stand der Technik bekannte, mit VP16 zusammenhängende Toxizität vermieden werden.

Die Aufgabe wird erfindungsgemäß durch einen rekombinanten viralen, insbesondere adenoviralen Vektor gelöst, der ein Insert enthält, das die allgemeine Struktur
tTA - Intron¹ - TK⁺ - TetO₇ - CMV⁺ - Intron² - Transgen
aufweist, wobei
- TetO₇: der heptamerisierte Tetracylin-Operator ist,
- TK⁺: der minimale Thymidin Kinase-Promotor ist,
- tTA: eine Nukleinsäuresequenz ist, die für ein Fusionsprotein aus dem durch Tetracyclin induzierbaren Repressorprotein und der transkriptionellen Aktivierungsdomäne des Herpes simplex Virus VP16 kodiert,
- CMV⁺: der minimale Cytomegalievirus-Promotor ist und
- Transgen: eine für ein nicht-virales Protein kodierende Nukleinsäuresequenz ist,
- Intron¹: eine beliebige nicht-kodierende Nukleinsäuresequenz mit einer Länge von 0 bis etwa 1000 bp ist und
- Intron²: eine beliebige nicht-kodierende Nukleinsäuresequenz mit einer Länge von 0 bis etwa 1000 bp ist.

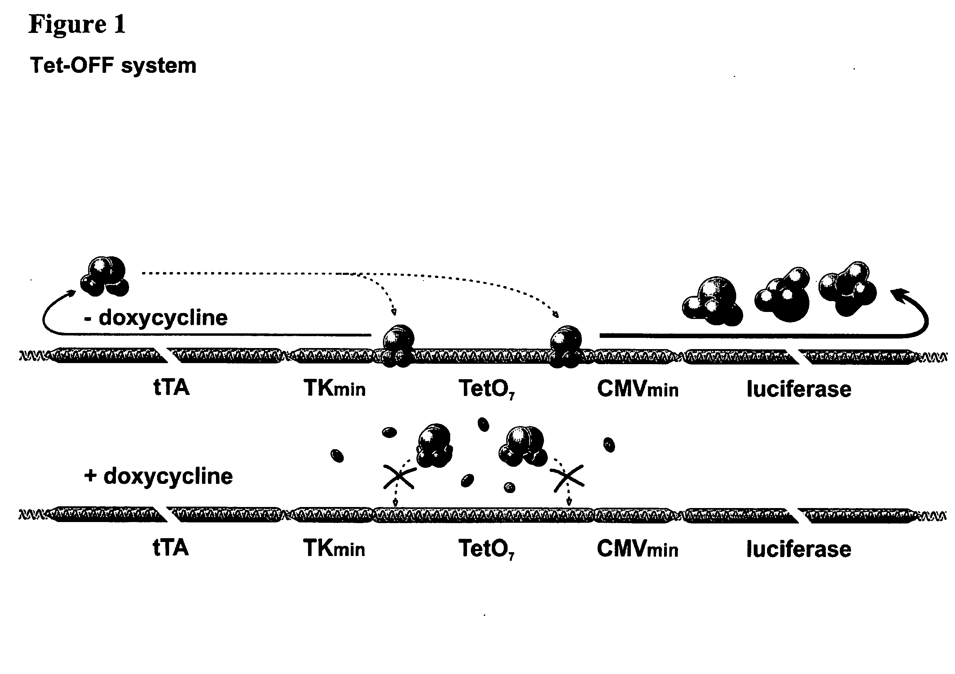

Für die Konstruktion der erfindungsgemäßen rekombinanten Adenoviren wurde das Tetracyclin induzierbare RepressorProtein (tetR) mit der transkriptionellen Aktivierungsdomäne des Herpes simplex Virus VP16 fusioniert. Im Vordergrund steht dabei nicht mehr die Inhibition durch Bindung des tetR an das Operon, sondern die Positionierung des VP16 Transaktivators. Entsprechend wurde für die vorliegende Erfindung auch ein heptamerisiertes TetO Operon mit zwei flankierenden Minimalpromotoren verwendet. Dieses System (Fig. 1) führt zur autoregulierten Transaktivator-Expression im Sinne eines positiven Feedback-Mechanismus über einen der Minimalpromotoren. Gleichzeitig wird ein therapeutisches Transgen über den anderen flankierenden Minimalpromoter exprimiert. Doxycyclin und Tetracyclin binden an die tetR-Komponente und eine Änderung der sterischen Konformation führt zu einem Verlust der Bindung des tetR an den Operator. Die Dissoziation des Transaktivators von den Minimalpromotoren hat dann eine Reduktion der Genexpression zur Folge.

Mit den erfindungsgemäßen Vektoren wurde erstmals ein replikationsdefizientes adenovirales System auf Basis eines Vektors erzeugt und charakterisiert, bei dem eine autoregulierte Transaktivator-Expression erfolgt. Dieses System ermöglicht eine sehr strenge Kontrolle der Transgen-Expression durch Zugabe von Doxycyclin in nicht-toxischen Konzentrationen. Die hohe Suppression der Genexpression wurde über einen weiten m.o.i.-Bereich (multiplicity of infection) und in verschiedenen Karzinomzellinien erreicht.

Der Anteil der Suppression hängt von der verwendeten Konzentration des Antibiotikums ab. Da eine maximale Suppression der Transgenexpression bereits bei Doxycyclin-Konzentrationen von 2 *µ*g/ml erzielt wurden, sind die erfindungsgemäßen Vektoren bestens zur klinischen Anwendung geeignet.

Gemäß einer weiteren Ausführungsform betrifft die Erfindung einen genannten Vektor, bei dem das Insert in umgekehrter Orientierung in das virale Vektorgenom inseriert ist, d.h. in der Form
5' -Transgen-Intron²-CMV⁺-TetO₇-TK⁺-Intron¹-tTA-3'.

Ebenso ist es möglich, daß ausschließlich oder zusätzlich die Positionen von tTA und Transgen im Insert vertauscht sind.

Soweit die Sequenzelemente 'Intron¹' und/oder Intron²' vorhanden sind (d.h. > 0 bp), kann deren Länge im Bereich bis zu etwa 1000 bp unabhängig voneinander variieren und z.B. jeweils bis etwa 750, bis etwa 500 oder bis etwa 250 bp betragen. In diesem Fall werden die Promotoren üblicherweise innerhalb der jeweiligen Intronsequenz liegen.

Gemäß einer weiteren Alternative kann das Insert zwischen 'CMV⁺' und 'Intron²' oder zwischen 'Intron²' und 'Transgen' zusätzlich einen lac-Repressor (lacR) enthalten, wodurch eine weitere Regulationsmöglichkeit geschaffen wird.

Das verwendete Transgen ist eine für ein Fluoreszenzprotein, für Luciferase, Interleukin-12 (IL-12), Interleukin-18 (IL-18), Interleukin-2 (IL-2), Tumor Nekrose Faktor α (TNF-α) oder Interferon-γ (IFN-γ) kodierende Nukleinsäuresequenz, vorzugsweise single-chain Interleukin-12. Die Erfindung betrifft ferner Vektoren, bei dem einer der flankierenden Promotoren zur Expression eines Genes zur Apoptose-Induktion, zur Expression des BAX Genes, zur Expression des FAS-L Genes, eines Suizid-Genes, wie Thymidin-Kinase-oder Cytosin-Deaminase-Gen, oder eines β-Galaktosidase-Genes verwendet wird.

Hinsichtlich des verwendeten Virusrückgrats eignet sich insbesondere ein Adenovirus, ein Adenoassoziiertes Adenovirus (AAV), ein Retrovirus, insbesondere ein Humanes Immundefizienzvirus (HIV), ein Herpes Simplex Virus, ein Hepatitis-B virus oder ein Hepatitis C-Virus, wobei Adenoviren besonders bevorzugt sind.

Bei dem erfindungsgemäßen Vektor ist das Insert in die E1-Region eines rekombinanten Adenovirus einkloniert, alternativ eignen sich auch die E3- und/oder die E4-Region.

Gemäß einer besonderen Ausführungsform betrifft die Erfindung einen Vektor, der zum Beispiel durch homologe Rekombination eines viralen, insbesondere eines adenoviralen Plasmids und eines Expressionsplasmids mit der in SEQ ID NO:1, SEQ ID NO:2 oder SEQ ID NO:3 dargestellten Nukleinsäuresequenz erhältlich ist. In diesem Zusammenhang steht "SEQ ID NO:" für die gemäß WIPO-Standard ST.25 verwendete Kennziffer <400>.

Gegenstand der Erfindung ist ferner ein Expressionsplasmid mit der in SEQ ID NO:4 oder SEQ ID NO:5 dargestellten Nukleinsäuresequenz sowie dessen Verwendung zur Herstellung eines oben genannten, erfindungsgemäßen Vektors.

Wie bereits erwähnt eignen sich die Vektoren zur in vitro-Genexpression in eukaryoten Zelllinien oder, wenn 'Transgen' für ein therapeutisch wirksames Protein kodiert, zur Herstellung eines Medikaments zur Gentherapie. Beispielsweise kann 'Transgen' IL-12 oder IL-18 sein, wodurch sich der Vektor zur Gentherapie maligner Erkrankungen eignet. Bei den malignen Erkrankungen handelt es sich insbesondere um einen soliden Tumor.

Bei den zuvor genannten Verwendungen wird die Genexpression mit Tetracyclin oder Tetracyclin-Derivaten, insbesondere mit Doxycyclin, Oxytetracyclin, Chlortetracyclin, Demeclocyclin, Methacyclin oder Minocyclin, reguliert. Soweit vorliegend Doxycyclin erwähnt ist, wird der Fachmann die Übertragbarkeit des erfindungsgemäßen Prinzips auf die oben genannten Tetracyclin-Derivate erkennen.

### Weitere Vorteile der Erfindung:

Im Vergleich zu einer durch den im Stand der Technik stark verwendeten konstitutiven humanen Zytomegalivirus (HCMV) immediate-early Promotor getriebenen Expression des IL-12-Heterodimers wird bei den im Rahmen der Erfindung entwickelten Konstrukten in einer großen Zahl von Krebszellinien eine bis zu 4000-fach höhere Zytokin-Sekretion beobachtet. Dieser unerwartete Effekt wird auf das Zusammenspiel aus der Wahl der Promotors und der Verwendung von genetisch erzeugtem und hoch-sekretorischem, einzelkettigem IL-12 zurückgeführt. Die IL-12-Expression in Abwesenheit von Doxycyclin war überraschenderweise ebenfalls den zuvor bereits veröffentlichten adenoviral infizierten murinen Tumorzellen unter Verwendung des CMV-Promotors zu Regulierung der Expression des heterodimeren oder einzelkettigen mIL-12 überlegen. Da die Transduktion humaner Tumorzellen im Vergleich zu zuvor berichteten Werten aus präklinischen oder klinischen Versuchen bei den erfindungsgemäßen Konstrukten ebenfalls signifikant höher ist, wird somit vorliegend ein weiterer, äußerst vorteilhafter Effekt erzielt. Durch die Möglichkeit, die adenovirale Dosis der erfindungsgemäßen Vektoren zu reduzieren, lassen sich nämlich Vektor-spezifische Nebeneffekte vermindern, was zu einer höheren Sicherheit in der klinischen Anwendung führt.

Im Hinblick auf die gemäß einer besonderen Ausführungsform genutzte Interleukin-12-Expression (siehe unten) haben die erfindungsgemäßen adenoviralen Vektoren ferner den Vorteil, daß sie alle Voraussetzungen zur erfolgreichen gentherapeutischen Krebsbehandlung erfüllen. So wird durch Expression des einzelkettigen Interleukin-12, das, verglichen mit der allgemein verwendeten heterodimeren Form, eine ähnliche Bioaktivität aufweist, die Bildung von inhibitorischen p40-Homodimeren vermindert, und durch die schnelle Regulation der Genexpression im erfindungsgemäßen 3r-System durch effiziente Sekretion des einzelkettigen Interleukin-12 sichergestellt. Die äußerst effiziente, Doxycyclin-vermittelte Suppression der Expression des bioaktiven, einzelkettigen Interleukin-12 trägt somit zur Sicherheit bei der gentherapeutischen Krebsbehandlung bei.

Das erfindungsgemäße System zeichnet sich ferner dadurch aus, daß eine westliche Standardernährung das sensitive tet-OFF-System nicht beeinflußt wird, so daß mögliche Belastungen der Nahrung mit Spuren an Tetracyclin oder dessen Derivaten im klinischen Umfeld kein Problem darstellen.

Durch die Verwendung der erfindungsgemäßen Vektoren ist die Anwesenheit von Transaktivatoren vor der Infektion mit den Vektoren nicht erforderlich, wodurch die Toxizität infolge der konstitutiven Expression des Transaktivators sowie eine gegenseitige Beeinflussung oder Störung der Transkription durch Doxycyclin-abhängige, autoregulative Genexpression vermieden wird. Demzufolge stellen die adenoviralen Vektoren der vorliegenden Erfindung ein wesentlich vielseitigeres und unaufwendigeres Hilfsmittel im Vergleich zu den im Stand der Technik bekannten Modellen der konstitutiven Transaktivator-Expression dar.

Ferner ist von Vorteil, daß die Doxycyclin-regulierte Genexpression nach adenoviraler Infektion einer großen Vielzahl nativer Säugerzelllinien oder Gewebe erfolgen kann. Die Autoregulation bewirkt ferner eine Beschränkung der ungewollten Transgen-Expression durch reduzierte Transaktivator-Expression bei Suppression durch Doxycyclin. Gegenüber den im Stand der Technik beschriebenen Ansätzen bieten die erfindungsgemäßen Vektoren den Vorteil, daß in Abwesenheit von Doxycyclin eine sehr hohe Transgen-Expression erreicht wird, während die Suppression der Transgen-Expression durch die Zugabe dieses Antibiotikums nicht beeinträchtigt wird, und bis zu 6000-fache Suppressions-Level erzielt werden.

Die erfindungsgemäßen Konstrukte lassen sich somit in vorteilhafter Weise zu Expression therapeutischer Transgene von bis zu 4,8 kB, einschließlich Apoptose-induzierender Gene, verwenden und stellen damit ein wichtiges Mittel zur molekularen Therapie maligner Erkrankungen dar.

Im Rahmen der vorliegenden Erfindung wurde ferner überraschenderweise festgestellt, daß die erfindungsgemäßen Vektoren eine mindestens 40-fach höhere Sensitivität gegenüber Tetracyclin aufweisen als die Detektionsgrenze in Standard-HPLC-Verfahren. Das erfindungsgemäße System eignet sich somit ferner als sensitiveres Hilfsmittel zum Nachweis sehr geringer Tetracyclinkonzentrationen in biologischen, lebensmittelchemischen oder ähnlichen Proben und ist somit beispielsweise zum Einsatz in der human- und veterinärmedizinischen Diagnostik geeignet (vgl. N. Schultze et al. Nat. Biotechnol. 14 (1996) 499-503). Das Transgen kodiert in diesem Fall für ein Reporterprotein, wie z.B. Luciferase oder ähnliches. Gegenstand der Erfindung ist somit ferner die Verwendung der erfindungsgemäßen Vektoren, bei denen 'Transgen' für ein Reporterprotein kodiert, zum Nachweis von Tetracyclin oder einem Derivat desselben, wie z.B. Doxycyclin, in biologischen, lebensmittelchemischen oder ähnlichen Proben.

Die Erfindung wird nachfolgend anhand von Beispielen näher erläutert.

### Beispiele

### Zellinien

HeLa und 293 humane embryonale Nierenzellen wurden in HGDMEM (Gibco, Rockville, MD) kultiviert. Humane RT-4 Blasenkrebszellen und humane Colon Adenokarzinomzellen HT29 wurden in McCoy-Medium (Gibco) gehalten. MCF-7 und BT-20 humane Brustkrebszellen sowie humane Colon (Colo 205 und SkCO-1) und pankreatische Adenokarzinom- (Aspc-1) Zellinien ließ man in RPMI-Medium (Gibco) wachsen. HepG2 humane hepatozelluläre Karzinomzellen wurden in MEM-Medium (Gibco) gehalten. Zellen wurden kultiviert und gemäß Standardvorschriften aufgeteilt. Alle Medien waren mit 10% fötalem Rinderserum (FBS), 1% Penicillin/Streptomycin (Gibco) und 1% Glutamin (Gibco) supplementiert. Die humane Myelomzellinie U266 ließ man in RPMI-Medium wachsen, das mit 15% FBS (Clontech) und 1% Penicillin/Streptomycin (Gibco) supplementiert war.

### Beispiel 1

### Plasmidkonstruktion

DNA-Fragmente wurden durch Agarose-Gelelektrophorese getrennt und aus der Agarose mit dem Gel extraction Kit (Qiagen, Valencia, CA) eluiert. DH5alpha-Zellen wurden zur Plasmidvermehrung eingesetzt. Plasmid-DNA wurde unter Verwendung eines modifizierten Protokolls für eine alkalische Lyse, gefolgt von einer Reinigung über eine kommerziell erhältliche Ionenaustauschsäule nach Angaben des Herstellers (Qiagen), präpariert. Vor der Transfektion wurden LPS-Verunreinigungen in den Plasmid-DNA-Präparationen durch eine Triton X-117 Extraktionsmethode (M. Cotton et al., Gene therapy 1 (1994) 239-246) reduziert. Das Plasmid pBIG 3r, das das autoregulierte tTA Expressionssystem enthält, ist vorbeschrieben (C.A. Strathdee, Gene 229 (1999) 21-29). Die Luciferase cDNA wurde aus dem Plasmid pGL3basic (Promega, Madison, WI) durch BglII- und XbaI-Verdau erhalten und in pBIG 3r inseriert, das mit SpeI und BamHI gespalten wurde, was zur Erzeugung von pBIG 3r luc führte. Die adenovirale Plasmid pAd.CMV-Expressionskassette wurde durch Verdau mit XbaI und SalI nach Auffüllen mit T4 DNA-Polymerase entfernt. PBIG 3r luc wurde mit PvuII und SalI verdaut, und die Fragmente, die die bicistronische Expressionskassette enthielten, wurden in das Rückgrat von pAd.CMV-pA ligiert. Das resultierende adenovirale Plasmid pAd3r-luc enthielt die bidirektionale Expressionskassette, die an ihrem 5'-Ende durch die 1-456 bp des AD5-Genoms einschließlich Linker ITR und Verpackungssignale flankiert ist und die an ihrem 3'-Ende durch 3346-5865 bp des AD5-Genoms flankiert ist. Die von minimalen TK-Promotor getriebene Expression des tTA war antiparallel, und die durch den minimalen CMV-Promotor getriebene Expression des Luciferase-Gens war parallel zur adenoviralen E1-Transkription. Das Luciferase-Gen wurde aus pGL3-basic durch Verdau mit KpnI/SalI freigesetzt und in das adenovirale Expressionsplasmid pAd.CMV-pA ligiert, was zu pAd.CMV-luc führte. Die cDNA des einzelkettigen murinen Interleukin-12 wurde aus pSFG.IL-12.p40.L.p35 (G.J. Lieschke et al. Nat. Biotechnol. 15 (1997) 35-40) nach Verdau mit NcoI und EcoRV erhalten. Dieses Fragement wurde in den NheI/SalI-Ort von pAd.3r-luc subkloniert und ersetzte das Luciferase-Gen. Das nachfolgend verwendete Plasmid pAd.CMV.p40.IRES.p35 enthält die zwei murinen IL-12 Untereinheiten, die durch eine Internal Ribosome Entry Site (IRES) des Enzephalomyocarditisvirus getrennt sind. Die Expression dieses Konstrukts steht unter der Kontrolle des humanen Zytomegalovirus (CMV) Promotor-Elements von -601 bis -14 relativ zum Transkriptionsstart.

### Beispiel 2

### Erzeugung und Amplifikation rekombinanter adenoviraler Vektoren

Rekombinate E1- und E3-deletierte Adenoviren wurden erhalten und Plaque-gereinigt nach Kalziumphosphat-vermittelter Cotransfektion von pAd.3r-luc, pAd.CMV-luc, pAd.3r-scIL-12 oder pAd.CMV.p40.IRES.p35 mit pBHG10 (AJ Bett et al., PNAS USA 91 (1994) 8802-8806). Die E1- und E3-deletierten Adenoviren wurden in 293-Zellen repliziert und durch CsCl-Zentrifugation wie zuvor beschrieben (FL Graham, Virology 54 (1973) 536-539) gereinigt. Die Titration der gereinigten Viren wurde mittels Plaque-Assay durchgeführt. Die resultierenden Titer für Ad.3r-luc, Ad.CMV-luc, Ad.3r-scIL12 und Ad.CMV-p40.IRES.p35 waren 1,0 x 10¹⁰ p.f.u./ml (plaque forming units pro ml), 7,5 x 10⁹ p.f.u./ml, 6,7 x 10⁹ p.f.u./ml und 8,0 x 10⁹ p.f.u./ml. Virale DNA wurde erhalten (Qiagen DANN Blood Kit) zur Sequenzanalyse, um die Insertion, die Transaktivator-Sequenz und die Orientierung zu bestätigen.

### Beispiel 3

### In vitro adenovirale Transfektion

HT29, Colo205, SkCO-1, AsPc-1, HepG2, MCF-7, BT-20, HeLa, RT4 und U266 Zellen wurden in Platten mit sechs und zwölf (U266) Kavitäten bei einer Konzentration von 1 x 10⁶ Zellen pro Kapität 6 Stunden vor der Transfektion gesät. Die größeren HeLa-, RT-4- und 293-Zellen wurden bei einer Konzentration von 5 x 10⁵ Zellen pro Kapität gesät. U266-Myelomzellen wurden in Suspensionskultur wachsen gelassen und infiziert. Gereinigte virale Partikel wurden in Medien ohne Supplementierung verdünnt, und die Zellen wurden 500 µl der geeigneten Virusverdünnung pro Kapität für 1 Stunde ausgesetzt. Nach Entfernung des infektiösen Überstands wurden vollständige Medien, die mit unterschiedlichen Konzentrationen von Doxycyclin supplementiert waren, zugefügt. Die Medien wurden alle 24 Stunden gewechselt.

### Beispiel 4

### Quantifizierung der Transgen-Expression

24 Stunden nach Infektion mit Ad.CMV-luc oder Ad.3r-luc wurden die Zellen mit 150 µl Zellkultur-Lysisreagens nach den Angaben des Herstellers (Promega) geerntet. Die Luciferase-Aktivität in 20 *µ*l Zell-Lysat wurde unter Verwendung eines Bertold LB9507 Luminometers und Luciferase-Assaysubstrat (Promega) gemessen. Die Standardkurven wurden unter Verwendung von rekombinanter Leuchtkäfer-Luciferase (Promega), das mit CCLR auf Konzentrationen von 1 pg/ml bis 300 ng/ml verdünnt war, erzeugt. Da die rlu bei höheren Konzentrationen ein Sättigungsprofil zeigen, wurde ein 2-Phasen exponentielles Assoziations-Kurvenfitting unter Verwendung des Prism Software-Pakets (GraphPad Software, Inc, San Diego, CA) durchgeführt. Die Proteinkonzentration wurde unter Verwendung des DC-Protein-Assaykits (BioRad, Hercules, CA) bestimmt.

Die Quantifizierung des einzelkettigen und heterodimeren mIL12 in zellfreiem Überstand nach adenoviraler Infektion von Tumorzellen wurde durch einen IL12 p70 ELISA (OptEIA^{™}, Pharmingen) durchgeführt, wobei gleiche Immunreaktivität und Molekulargewicht für beide Formen angenommen wurde. Splenozyten wurden mit Hilfe von Standardverfahren isoliert. Gehirnzellen wurden dann für drei Tage mit RPMI 1649 kultiviert, das mit 10% FBS, 1% Penicillin/Streptomycin und 1% Glutamin in anti-Maus CD3-beschichteten Kolben in Gegenwart von anti-human CD28 (5µg/ml) kultiviert, um T-Zellen anzureichern und die mIL-2-Sekretion zu stimulieren. Die Bioaktivität wurde nach Zugabe von 50-fach verdünntem konditionierten Überstand aus Ad.3r-scIL12 (+/- Doxycyclin), Ad.CMV-p40.IRES.p35 und mock-infizierten HT29-Zellen zu 4 x 10⁴ murinen Splenozyten in einem finalen Volumen von 125 µl für 24 Stunden bestimmt. Murines IFN-γ wurde in Splenozytenfreiem Überstand unter Verwendung eines IFN-γ ELISA (OptEIA^{™}, Pharmingen) quantifiziert. Um die spezifische Bioaktivität zu bestimmen, wurden halblogarithmische Verdünnungen von konditioniertem Überstand von beiden Formen des adenoviral exprimierten mIL-12 und Baculovirus-exprimiertem, gereinigtem mIL12 (R&D systems) auf mIL-12-Immunreaktivität (p70 ELISA) und IFN-γ-Induktion in Splenozyten wie beschrieben getestet. Die Bioaktivität von adenoviral exprimiertem heterodimerem IL-12 kann durch die Bildung inhibitorischer p40-Homodimerer wie anderweitig beschrieben reduziert werden. Es wurde vorliegend kein Capture-Bioassay verwendet, um potentiell niedrigere Bioaktivität in vivo zu reflektieren.

### Beispiel 5

### Alternative Klonierungsstrategie

### Klonierung von adenoviralen Expressionsplasmiden für die Virussynthese mittels AdEasy System

Als Alternative zu der beschriebenen Virussynthese wurden adenovirale Expressionsplasmide entwickelt, die eine Virusgeneration mittels AdEasy® -System (Stratagene) ermöglichen. Dazu wurde der pShuttle Vektor (Stratagene) mit KpnI verdaut, gebluntet, und nachfolgend mit SalI verdaut. Das 3r-Insert wurde aus pBIG3r mittels Verdau mit PvuII und SalI isoliert und in den pShuttle ligiert. Das resultierende Plasmid pShuttle3r ermöglicht die einfache Generation verschiedener adenoviraler Vektoren zur Doxyzyklinsupprimierbaren Genexpression. Das humane Single-Chain Interleukin-12 kann nachfolgend mittels XhoI in die multiple cloning site des pShuttle3r kloniert werden und resultiert in pShuttle3r-hscIL12 (siehe Abbildung).

Die Virusgeneration erfolgt dann durch homologe Rekombination mit pAdEasy-1® in BJS183 E. coli-Zellen und Selektion für Kanamyzin. Nach Transfektion von 293 Zellen mit dem Rekombinationsprodukt entstehen in diesem System replikative rekombinante adenovirale Vektoren (T He, S Zhou et al. Proc Natl Acad Sci USA 95 (5): 2509-14).

Die Virusproduktion erfolgt dann wie vorbeschrieben in 293-Zellen.

### Beispiel 6

### Natriumdodecylsulfat-Polyacrylamid-Gelelektrophorese (SDS-Page) und Immunblotting

Nach Infektion von HT29-Colon Krebszellen wurden Lysate auf 15% Acrylamid SDS-Gele nach Kochen in Laemmli-Probenpuffern unter reduzierenden Bedingungen geladen. Nach elekrophoretischer Auftrennung wurden die Proteine auf 0,45 µm Immobilon-P (Millipore, Bedford, MA) transferiert und mit TBS-enthaltender 5% nicht-fetter Trockenmilch für 1 Stunde geblockt. Actin und das Fusionsprotein tTA wurden unter Verwendung eines Kaninchen anti-Actin Affinitäts-isolierten Antigen-spezifischen Antikörpers (#A2066, Sigma, St. Louis, MS) und eines Maus anti-TetR monoklonalen Antikörpers (M. Gossen et al., PNAS USA 89 (1992) 5547-5551) (#8632-1, Clontech) nachgewiesen. Nach Inkubation für 1 Stunde wurden die Blots mit TBS-enthaltendem 0,1% Tween-20, pH 7,5, gewaschen und mit anti-Kaninchen und anti-Maus Peroxidaseverknüpften sekundären Antikörpern (Dianova, Hamburg, Deutschland) für 1 Stunde bei Raumtemperatur inkubiert. Proteine wurden schließlich nach Waschen und Chemolumineszenz-Nachweis (SA Nesbitt et al. Anal. Biochem. 206 (1992) 267-272) (ECL, Amersham, Buckinghamshire, UK) nach Vorschrift des Herstellers visualisiert.

### Beispiel 7

### Tetracyclin-Screening von Blutspender-Seren

Eine Frau und sieben Männer im Alter zwischen 23 und 35 Jahren wurden als Probanden ausgewählt. Sie hatten für mindestens einen Monat keine antiinfektiöse Behandlung erhalten. Alle waren gesund und unter einer westlichen Standardernährung. 50 ml periphären venösen Bluts wurden entnommen, und das Serum wurde nach Standardverfahren erhalten. Die Seren unterliefen einem Gefrier-Auftau-Zyklus, bevor die Zellkultur-Experimente und Tetracyclin-Bestimmungen durchgeführt wurden. Humane Seren wurden anstelle von FBS den Zellkulturmedien zugegeben. Tetracyclin-HCl wurde von Fluca Chemicals (Fluca, Germany) gekauft. Bakerbond RP-18 Festphasenextraktions (SPE)-Säulen wurden von Mallinckrodt Baker (Phillipsburg, NJ) erhalten, Lösungsmittel in HPLC-Qualität und andere Chemikalien wurden von Merck (Whitehouse Station, NJ) gekauft. HPLC wurde an Constametric 3500 MS und RP-18 HYPURITY® ADVANCE-Säulen von ThermoQuest (Deutschland) durchgeführt. Die Datenanalyse erfolgte unter Verwendung von Chemstation Software von Agilent (Deutschland) nach Vorkonditionierung der RP-18-Säulen mit 2 x 1 ml Methanol, gefolgt von 2 x 1 ml Wasser, wurden 3 ml Serum, das 0,1 mol/l Citratpuffer (pH 6, 8) und 0, 1 mol/l EDTA enthielt, bei einer Fließgeschwindigkeit von 1 ml/min. zugegeben. Die Säulen wurden dann mit 10 ml Wasser und 1 ml Methanol gewaschen. Tetracyclin wurde mit 4 ml Methanol, das 0,1% Trifluoressigsäure enthielt, eluiert (ME Sheridan et al. J. Chromatography 434 (1988) 253-258). Das Eluat wurde getrocknet und in 100 µl 0,01% Oxalsäure in Wasser/Acetonitril (98/2 v/v) bei einem mit HCl eingestellten pH-Wert von 2,0 rekonstituiert. Die Chromatographie wurde bei Raumtemperatur und einer Fließgeschwindigkeit von 0,9 ml/min. durchgeführt. Die Fluoreszenz bei 416 nm (Anregung) und 515 nm (Emission) wurde durch Komplexierung des Tetracyclins mit 0,2% (w/v) Zirkonium(IV)chlorid erreicht (K. De Wasch et al. Analyst 123 (1998) 2737-2741). Die Kalibrierung wurde mit wässrigen Lösungen von Tetracyclin HCl von 2 bis 100 ng/ml mit Variationskoeffizienten von 6,3% (während eines Tages) und 8,5% (von einem Tag auf den anderen) für 10 ng/ml durchgeführt.

### Resultate

### Konstruktion von Doxycyclin-supprimierbaren, auto-regulierten adenoviralen Vektoren

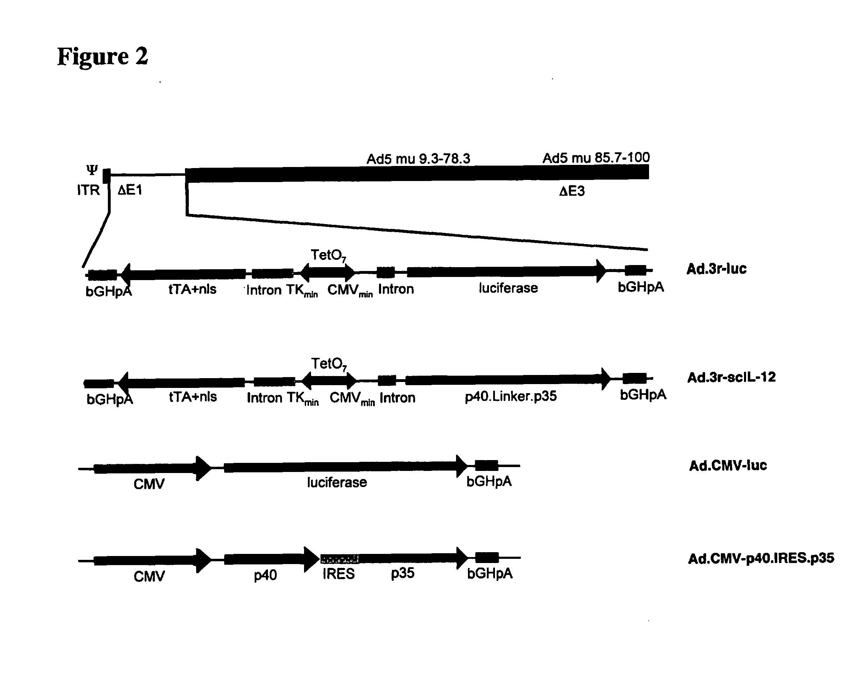

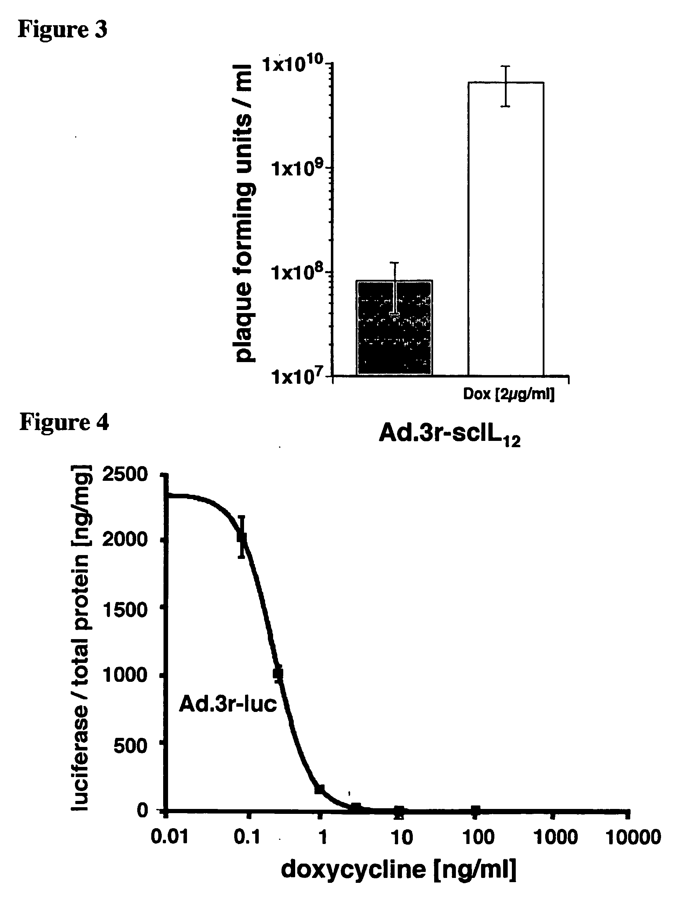

Adenovirale Expressionsplasmide, die das Luciferase- und murine scIL-12-Gen unter Kontrolle des Tetracyclinsupprimierbaren autoregulierten Systems enthielten, pAd.3r-luc und pAd.3r-scIL-12 wurden erzeugt. Nach einem gleichen Verfahren wurden Plasmide erzeugt, die das Luciferase-Gen und die cDNA, die für murines p40 und p35, das durch einen Internal Ribosome Entry Site (IRES), jeweils unter Kontrolle des Zytomegalivirus (CMV)-Promotors, enthalten. Rekombinate El/E3-deletierte Adenoviren Ad.3r-luc, Ad.3r-scIL12, Ad.CMV-luc und Ad.CMV-p40.IRES.p35 (Figur 2) wurden durch Cotransfektion adenoviraler Expressionsplasmide mit pBHG10 erzeugt. Die Plaque-Reinigung und Amplifizierung wurde in 293-Zellen durchgeführt. Adenovirale Titer wurden durch Standard-Plaque-Assay-Techniken quantifiziert. Isolierung, Amplifikation und Plaque-Assay von Ad.3r-scIL12 war in Gegenwart von 2 *µ*g/ml Doxycyclin bis zu 87-fach höher, was auf die Toxizität nicht supprimierter scIL12-Expression in 293-Zellen hinweist (Figur 3). Im Gegensatz dazu hatte Doxycyclin keinen Einfluß auf die Titrierung von Ad.3r-luc.

### Dosis-abhängige Doxycyclin-regulierte Luciferase- und Transaktivator-Genexpression

Humane Colon-Krebszellen HT29 sind gegenüber adenoveraler Transduktion äußerst empfänglich, wie bereits zuvor gezeigt (A. Block et al. Cancer Gene Therapy 7 (2000) 438-445). Diese Zellen wurden mit Ad.3r-luc bei einer m.o.i. (multiplicity of infection) von 30 nach Inkubation mit Doxycyclin bei verschiedenen Konzentrationen für 24 Stunden infiziert. Die Luciferase-Aktivität wurde in Zelllysaten entsprechend dem gelösten Zellprotein bestimmt. Bereits geringe Doxycyclin-Konzentrationen wie 100 pg/ml führen zu einer signifikanten Reduktion der Genexpression. Die Genexpression wurde schließlich mit Doxycylcin-Konzentrationen von bis zu 3 µg/ml maximal supprimiert (Figur 4). Diese Doxycyclin-Konzentration wird üblicherweise in der klinischen Behandlung bakterieller Infektionen verwendet. Im vorliegenden experimentellen Ansatz lag eine bis zu 2400-fache Doxycyclin-vermittelte Suppression der Transgen-Expression vor.

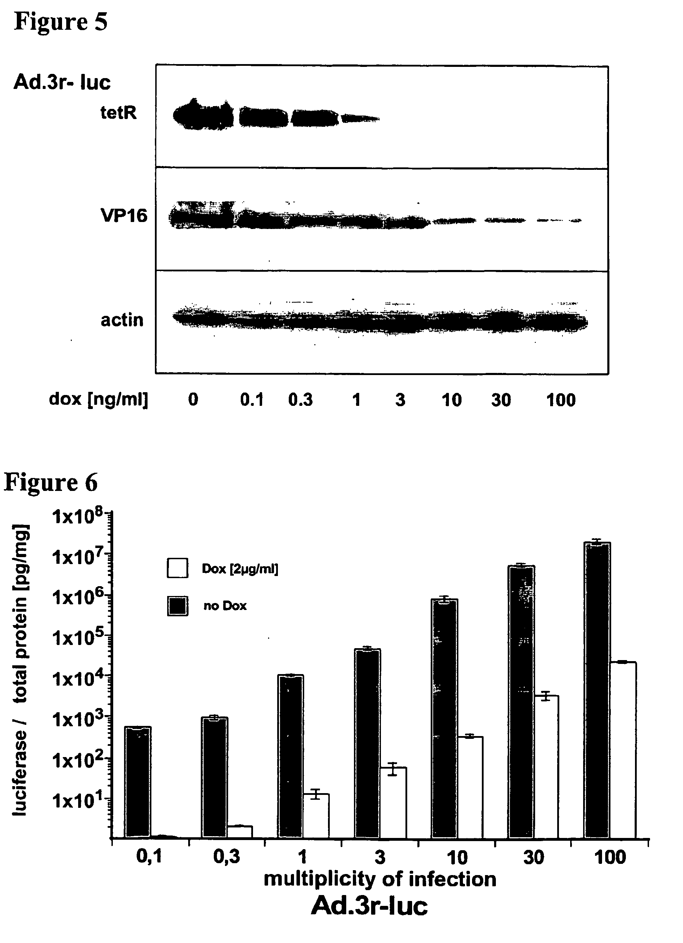

Die dosis-abhängige, Doxycyclin-regulierte Supression der positiven Feedback-Schleife (Figur 1) wurde durch Nachweis der tTA Fusionsproteine mit Tet-R monoklonalen (M. Gossen et al., PNAS U.S.A. 89 (1992) 5547-5551) und VP16 polyklonalen Antikörpern (PE Pellett et al. PNAS U.S.A. 82 (1985) 5870-5874) in Western Blot-Analysen illustriert (Figur 5). Steigende Doxycyclin-Konzentrationen führten zu einer Herunterregulierung der intrazellulären tTA-Anteile, was mit einer verminderten Luciferase-Genexpression korreliert.

### M.O.I.-abhängige, supprimierbare Luciferase-Expression

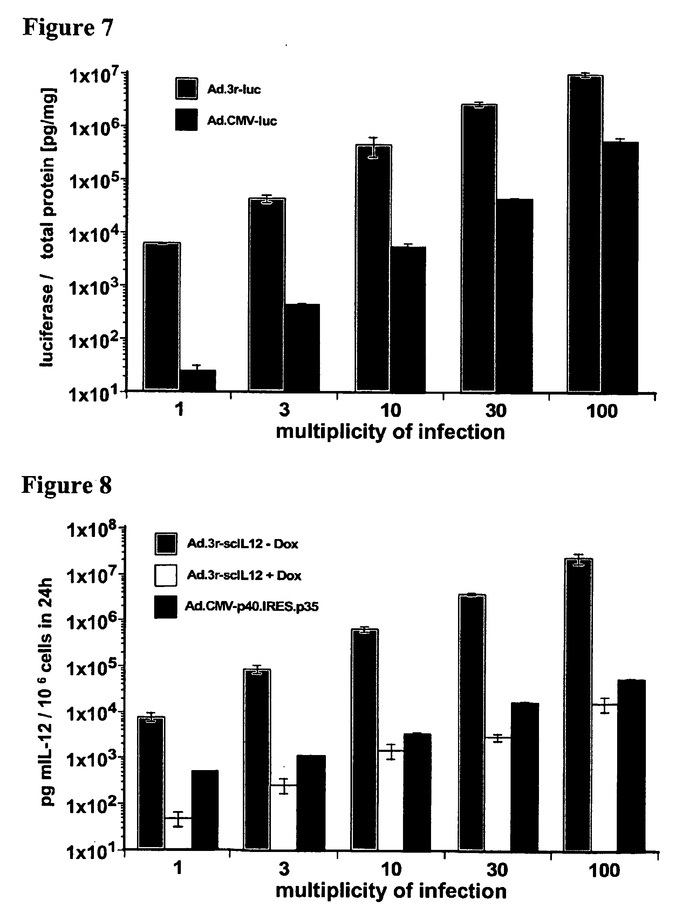

HT29-Zellen wurden mit Ad.3r-luc bei einer m.o.i. im Bereich von 0,1 bis 100 nach Inkubation in Gegenwart oder Abwesenheit von Doxycyclin bei 2 µg/ml für 24 Stunden infiziert. Die Suppression der Luciferase-Gen-Expression in Lysaten von Ad.3r-luc-infizierten HT29-Zellen bewegte sich von 470 (m.o.i.: 0,3) bis 2400-fach (m.o.i.: 10 - 100) (Figur 6). Das Ausmaß der Suppression blieb bei hohen m.o.i. konstant, was für eine ausreichende, mit der Transgen-Expression zusammenhängende Toxizitätskontrolle entscheidend ist. Doxycyclinkonzentrationen von 2 *µ*g/ml störten die adenovirale Genexpression in HT29-Zellen unter Verwendung des konstitutiven CMV-Promotors nicht. Um die Effizienz der Ad.3r-luc-vermittelten transgenen-Expression in Abwesenheit von Doxycyclin zu untersuchen, wurde die Expression mit der Expression in HT29-Zellen verglichen, die mit Ad.CMV-luc infiziert waren (Figur 7). In HT29 zeigte Ad.3r-luc eine höhere Genexpression als Ad.CMV-luc über alle getesteten m.o.i. (1 - 100), wobei der Faktor zwischen 18-fach (m.o.i.:100) und 240-fach (m.o.i.:1) lag.

### M.O.I.-abhängige regulierte Expression des einzelkettigen murinen Interleukin-12

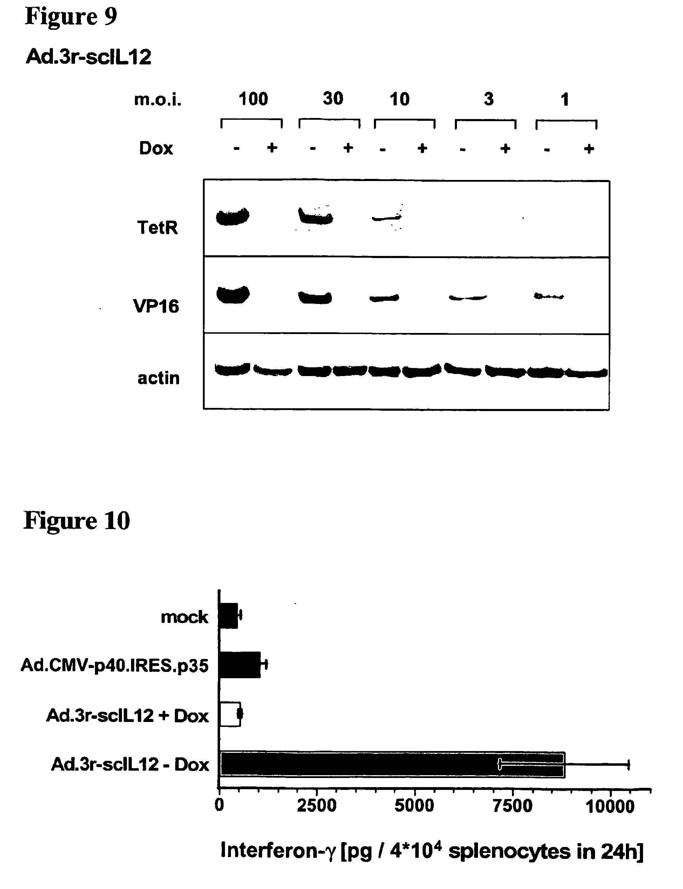

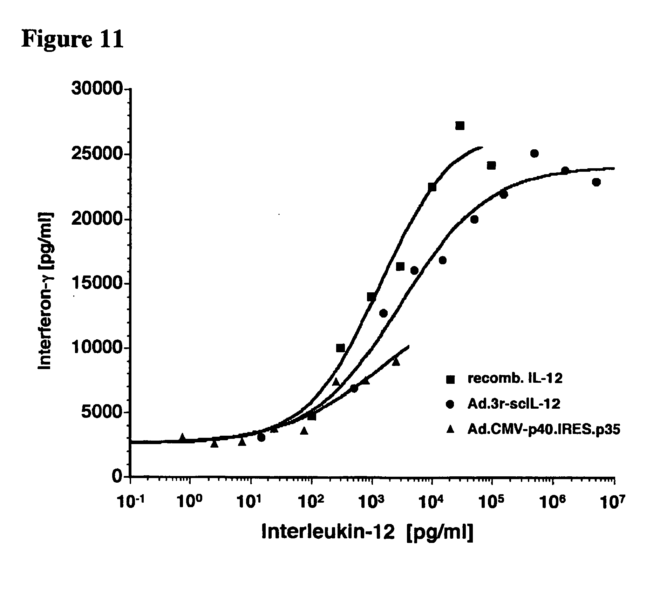

HT29-Zellen wurden mit Ad.3r-scIL-12 bei einer m.o.i. im Bereich von 1 bis 100 infiziert und in Gegenwart oder Abwesenheit von 2 *µ*g/ml Doxycyclin für 24 Stunden inkubiert. Die Genexpression von ScIL-12 wurde um mehr als 1400-fach bei einer m.o.i. von 100 in Gegenwart von Doxycyclin suprimiert (Figur 8). Die Western Blot-Analyse zeigte eine mit der IL-12 Expression korellierende Transaktivator (tTA)-Expression (Figur 9). Nicht-supprimierte 3r-vermittelte IL-12 Genexpression war 11-fach (m.o.i.:1) bis 375-fach (m.o.i.:100) höher als unter Verwendung des konstitutiven CMV-Promotors, wobei von gleicher Immunreaktivität des p70-ELISA gegenüber dem einzelkettigen Interleukin-12 und der CMV-gesteuerten Expression eines p40/p35-Heterorotrimers ausgegangen wurde. Die Bioaktivität beider Formen wurde durch Inkubation muriner Splenocyten mit 50-fach verdünnten konditionierten Medien nach Infektion von HT29 mit IL-12 exprimierenden Adenoviren quantifiziert (Figur 10). Es wurde eine hohe Interferon-γ (IFN-γ)-Sekretion der Splenocyten durch Inkubation mit konditionierten Medien nach Infektion von HT29 mit Ad.3r-sc-IL-12 erhalten. Diese IFN-γ-Induktion war verglichen mit der Infektion von HT29 mit dem gewöhnlich verwendeten Ad.CMV-p40.IRES.p35 signifikant höher. Die Zugabe von Doxycyclin resultierte in einer Suppression von IFN-γ auf einen Hintergrund-Level. Ferner wurde die spezifische Bioaktivität adenoviral exprimierter Formen von IL-12 im Vergleich zu rekombinant gereinigten p40/p35-Heterodimeren analysiert (Figur 11). Murine Splenocyten wurden mit halblogarithmischen Verdünnungen von rekombinant heterodimerem IL-12 oder konditionierte Medien enthaltendem IL-12 wie beschrieben inkubiert. Die IFN-γ-Induktion korellierte mit der Immunreaktivität von IL-12 in den Medien, wie durch p70-ELISA nachgewiesen wurde. Die basale Induktion wurde durch Präinkubation von Splenocyten mit Anti-humanen CD28-Antikörpern verursacht, was zur IL-2-Expression und nachfolgenden IFN-γ-Induktion führte (CH June et al., J. Immunol. 143 (1989) 153-161). Die Bioaktivität des murinen einzelkettigen IL-12 Fusionsproteins war mit dem gereinigten, rekombinanten p40/p35-Heterodimer vergleichbar. Die reduzierte Bioaktivität von IL-12, das nach Infektion mit dem gewöhnlich verwendeten Ad.CMV-p40.HRES.p35 exprimiert wurde, erklärt sich durch inhibitorische p40-Homodimere (P. Ling et al J. Immunol. 154 (1995) 116-127; S. Gillesen et al. European J. Immunol. 25 (1995) 200-206; F. Mattner et al. European J. Immunol. 23 (1993) 2202-2208).

### Regulierte Interleukin-12 Genexpression in vitro

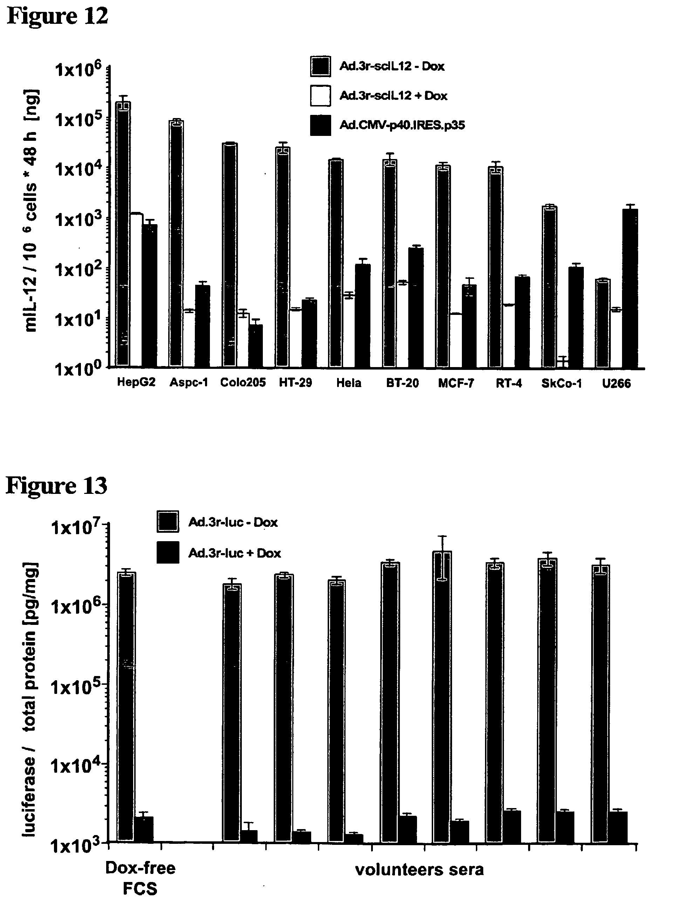

Verschiedene Zellinien des humanen Colon Karzinom (HT29, SkCo-1 und Colo205), pankreatischen Karzinom (Aspc-1), Blasen-Karzinom (RT4), Cervix-Karzinom (HeLa), Brust-Karzinom (MCF-7 und BT-20) sowie Myolom (U266) und hepatozelluläres Karzinom (HepG2) wurden entweder mit Ad. CMV.p40.IRES.p35 oder Ad.3r-scIL-12 infiziert und in Gegenwart oder Abwesenheit von Doxycyclin inkubiert. Die Expression des rekombinanten Interleukin-12 wurde unter Verwendung eines p70-ELISA wie zuvor beschrieben bestimmt (Figur 12). Die Doxycyclin-vermittelte Suppression der Interleukin-12-Expression trat in allen Zelllinien auf. In Abwesenheit von Doxycyclin erwies sich der 3r-Promotor gegenüber dem CMV-Promotor in allen Zelllinien mit Ausnahme der U266-Mylomzellinie als überragend. Die Interleukin-12-Expression in Mock-transfizierten Zellinien wurde nicht nachgewiesen. Die Suppression des IL-12 war 3,9-fach in U266 und bewegte sich von 167 (HepG2) bis 6000-fach (Aspc-1). Mit Ausnahme von U266, wo eine gegenüber der CMV-vermittelten IL-12-Expression signifikant geringere 3r-vermittelte IL-12 Expression vorlag, führte der 3r-Promotor in allen anderen Krebszelllinien zu 17-fach (SkCo-1) bis 4254-fach (Colo205) höherer Genexpression in Abwesenheit von Doxycyclin.

### Regulierte Genexpression nach Inkubation mit Humanserum

In Abetracht der geringen Doxycyclin- und Tetracyclin (tet)-Konzentrationen, die zur Suppression der Transgen-Expression erforderlich sind, wurde die Regulation in humanen Colon-Krebszellen in Gegenwart von humanem Serum untersucht, um die Anwendbarkeit dieses Ansatzes in einem möglichen klinischen Umfeld zu prüfen. Serumproben von gesunden Probanden, die sich einer westlichen Standardernährung unterzogen, wurden unter Verwendung eines Standard HPLC-Verfahrens mit einer maximalen Sensitivität von 2 ng/ml auf Tetracyclin getestet, da Tetracyclin in der Nutztierhaltung breite Anwendung findet und eine Kontamination von Nahrungsmitteln anzunehmen ist. Die HPLC zeigte keine signifikanten Tetracyclin-Konzentrationen in allen getesteten Proben. HT29-Colon-Karzinomzellen, die nach Inkubation mit diesen humanen Seren mit Ad.3r-luc (m.o.i.: 30) infiziert wurden, zeigten keinen signifikanten Unterschied hinsichtlich der Transgenexpression verglichen mit zertifiziertem, Tetracyclin-freiem fötalem Rinderserum (Figur 13). Diese Beobachtung spiegelt Tetracyclin-Konzentrationen in humanen Serumproben von weniger als 10 pg/ml wieder. Wie erwartet, führte die Supplementierung dieser Humanseren mit Doxycyclin (2 *µ*g/ml) zu einer äußerst effizienten Suppression der Transgen-Expression.

### Beschreibung der Figuren

**Figur 1.** Prinzip einer autoregulierten, Tetracyclinabhängigen Transaktivator-Expression. Der bidirektional tet-responsive Promotor kontrolliert sowohl das Transgen als auch die Transaktivator-Expression. Bindung des Transaktivators in Abwesenheit von Tetracyclin oder Doxycyclin resultiert in einer Amplifikation der Transaktivator-Expression durch eine positive Feedback-Schleife, ebenso wie in einer Induktion der Transgen-Expression. tTA, Tet-Repressor und VP16 Fusionsproteine; TKmin, minimaler Thymidin-Kinase-Promotor; CMVmin, minimaler Zytomegalovirus-Promotor; TetO₇, heptamerisierter Tet-Operator.

**Figur 2.** Adenovirale Vektorkarten. Die autoregulierte Tetracyclin-Expressionskassette ist in die ΔE1-Region des adenoviralen Genoms inseriert. Zur Vermeidung von kryptischem Splicing und um RNA-Stabilität zu erhalten (Ad.3r-luc und Ad.3r-scIL12) wurde ein Intron aufwärts vom Aktivator und dem Luciferase- oder Interleukin-12-Gen aus der Maus eingefügt. Zusätzlich wurden rekombinate adenovirale Vektoren für die Expression des Luciferase- oder des heterodimeren Interleukin-12-Gens der Maus unter der Kontrolle des CMV-Promotors konstruiert (Ad.CMV-luc und Ad.CMV-p40.IRES.p35). E1 und E3, Early regions des adenoviralen Genoms; IRES, interne Ribosomen-Eintrittsstelle; CMV, Zytomegalievirus-Promotor; TK, Thymidin-Kinase-Promotor.

**Figur 3.** Plaque-Assay von Ad.3r-scIL12 in An- und Abwesenheit von Doxycyclin in einer Konzentration von 2 *µ*g/ml. Titration von Ad.3r-scIL12 in 293-Zellen resultiert in einer erheblich höheren Ausbeute, wenn die Expression des Transgens durch Zugabe von Doxycyclin unterdrückt wird. Dox, Doxycyclin.

**Figur 4.** Dosisabhängige Luciferase-Expression nach Infektion von HT29 Dickdarmkrebszellen mit Ad.3r-luc gefolgt von verschiedenen Konzentrationen des Tetracyclin-Derivats Doxycyclin.

**Figur 5.** Westernblot-Analysen des Transaktivators zeigen die positive Rückkoppelungsschleife nach adenoviraler Infektion von HT29-Zellen unter Inkubation mit unterschiedlichen Mengen von Doxycyclin. Die Figur zeigt die Unterdrückung der Expression des tTA Fusionsproteins in Anwesenheit von Doxycyclin. dox, Doxycyclin.

**Figur 6.** Unterdrückung der Luciferase-Genexpression nach Infektion von HT29-Zellen mit verschiedenen Multiplizitäten der Infektion (multiplicities of infection, m.o.i.). Doxycyclin-regulierte Genexpression wird in einem großen Infektionsbereich von wenigstens 0,1 bis 100 m.o.i. erzielt, die in einer 470- bis 2400-fachen Unterdrückung der Luciferase-Expression resultiert.

**Figur 7.** Vergleich der 3r-vermittelten Transgen-Expression bei Verwendung des konstitutiven Zytomegalievirus-Promotors. HT29-Zellen wurden mit Ad.3r-luc oder Ad.CMV-luc in verschiedenen m.o.i. infiziert, gefolgt von einer Inkubation in Doxycyclin-freiem Medium.

**Figur 8.** Interleukin-12-Expression in HT29-Zellen nach Infektion mit Ad.3r-scIL12 in An- oder Abwesenheit von Doxycyclin (2 *µ*g/ml) oder Ad.CMV-mIL12 bei verschiedenen m.o.i. Wie mit Luciferase-exprimierenden adenoviralen Vektoren gezeigt wurde, befindet sich in HT29 eine erheblich höhere Interleukin-12-Expression, wenn der 3r-Promotor verwendet wird. Zugabe von Doxycyclin führt zu einer Unterdrückung der Transgen-Expression unter das Niveau, das mit Ad.CMV-p40.IRES.p35 bei der selben m.o.i. erzielt wird.

**Figur 9.** Westernblot-Analyse der tTA Transaktivator-Genexpression in An- oder Abweseneheit von Doxycyclin nach Infektion mit Ad.3r-scIL12 bei verschiedenen m.o.i.. Beide Domänen des tTA Fusionsproteins wurden mit den TetT- und VP16-Antikörpern nachgewiesen. Die Expression des tTA Fusionsproteins korreliert mit der eingesetzten m.o.i.. Zugabe von Doxycyclin in einer Konzentration von 2 *µ*g/ml resultiert in einer Unterdrückung der tTA Expression. TetR, Tetracyclin-Repressor; VP16, Herpes simplex Virus transkriptionale Aktivierungsdomäne.

**Figur 10.** Induzierung der Interferon-γ-Expression nach Inkubation von Splenozyten mit konditioniertem Überstand von infizierten HT29-Zellen. 10⁶ HT29-Zellen wurden mit Ad3r-scIL12 (+/- Dox) oder Ad.CMV-p40.IRES.p35 bei einer m.o.i. von 30 für 24h infiziert. Infektion von HT29 mit Ad.3r-scIL12 resultiert in einer starken Interferon-γ-Induktion im Vergleich zu einer Infektion mit Ad.CMV-p40.IRES.p35. Zugabe von Doxycyclin resultiert in einer Abnahme des Interferon-γ auf Hintergrundniveau in diesem Versuchsansatz.

**Figur 11.** Vergleich der Interferon-γ-Induktion durch adenoviral-exprimiertes Einzelketten- (single-chain), bzw. heterodimeres Interleukin-12, sowie durch aufgereinigtes rekombinantes Interleukin-12. Interleukin-12 im konditionierten Überstand von infizierten HT29-Zellen wurde durch p70-mIL12 ELISA bestimmt. Mäuse-Splenozyten wurden dann mit seriellen Verdünnungen von entweder adenoviral exprimierten oder rekombinanten Interleukinen inkubiert und das induzierte Interferon-γ wurde mit mIFN-γ ELISA quantifiziert. Die Bioaktivität-Immunoreaktivität von Einzelketten-Interleukin-12 war vergleichbar zu rekombinantem aufgereinigtem heterodimerem Interleukin-12. Die spezifische Bioaktivität von adenoviral produziertem heterodimerem Interleukin-12 (Ad.CMV-p40.IRES.p35) scheint niedriger zu sein, wahrscheinlich durch inhibitorische p40 Homodimere.

**Figur 12.** Interleukin-12-Expression in verschiedenen Zellinien nach Infektion mit entweder Ad.CMV-p40.IRES.p35 oder Ad.3r-scIL12 in An- oder Abwesenheit von Doxycyclin. Unterschiedliche Stärken der Transgenexpression beruhen z.T. auf Unterschieden in der Transduktionseffizienz. Mit Ausnahme der U266 Myelomzellinie war die 3r-vermittelte Genexpression deutlich höher als die CMV-vermittelte Expression.

**Figur 13.** Inkubation von Ad.3r-luc infizierten HT29 Dickdarmkarzinomzellen mit menschlichen Seren anstatt zertifiziertem Tetracyclin-freien fötalem Rinderserum. Es gab keine signifikanten Unterschiede bei der Verwendung von menschlichem Serum von Probanden mit einer standardisierten westlichen Ernährung im Vergleich mit zertifiziertem Tetracyclin-freiem fötalem Kälberserum. Diese Daten legen eine Tetracyclin-Konzentration bei den menschlichen Probanden von unter 50 pg/ml nahe. Die Ergänzung der menschlichen Sera mit Doxycyclin (2 *µ*g/ml) resultiert in einer Unterdrückung der Transgen-Expression wie vorgehend gezeigt. FCS, fötales Kälberserum.

## Patentansprüche

1. Rekombinanter viraler Vektor, der ein Insert enthält, das die allgemeine Struktur
tTA - Intron¹ - TK⁺ - TetO₇ - CMV⁺ - Intron² - Transgen
aufweist, wobei
TetO₇ der heptamerisierte Tetracyclin-Operator ist,
TK⁺ der minimale Thymidin Kinase-Promotor ist,
tTA eine Nukleinsäuresequenz ist, die für ein Fusionsprotein aus dem durch Tetracyclin induzierbaren Repressorprotein und der transkriptionellen Aktivierungsdomäne des Herpes simplex Virus VP16 kodiert,
CMV⁺ der minimale Cytomegalievirus-Promotor ist und
Transgen eine für ein nicht-virales Protein kodierende Nukleinsäuresequenz ist,
Intron¹ eine beliebige nicht-kodierende Nukleinsäuresequenz mit einer Länge von 0 bis etwa 1000 bp ist und
Intron² eine beliebige nicht-kodierende Nukleinsäuresequenz mit einer Länge von 0 bis etwa 1000 bp ist.

2. Vektor nach Anspruch 1, **dadurch gekennzeichnet, daß** das Insert in umgekehrter Orientierung in das virale Vektorgenom inseriert ist.

3. Vektor nach Anspruch 1 oder 2, **dadurch gekennzeichnet, daß** die Positionen von tTA und Transgen im Insert vertauscht sind.

4. Vektor nach den Ansprüchen 1 bis 3, **dadurch gekennzeichnet, daß** das Insert zwischen 'CMV⁺' und 'Intron²' oder zwischen 'Intron^{2'} und 'Transgen' zusätzlich einen lac-Repressor (lacR) enthält.

5. Vektor nach den Ansprüchen 1 bis 4, **dadurch gekennzeichnet, daß** das Transgen eine für ein Fluoreszenzprotein, für Luciferase, Interleukin-12 (IL-12), Interleukin-18 (IL-18), Interleukin-2 (IL-2), Tumor Nekrose Faktor α (TNF-α) oder Interferon-γ (IFN-γ) kodierende Nukleinsäuresequenz ist.

6. Vektor nach Anspruch 5, **dadurch gekennzeichnet, daß** IL-12 ein single-chain Interleukin-12 ist.

7. Vektor nach den Ansprüchen 1 bis 6, **dadurch gekennzeichnet, daß** das Virus ein Adenovirus, ein Adenoassoziiertes Adenovirus (AAV), ein Retrovirus, insbesondere ein Humanes Immundefizienzvirus (HIV), ein Herpes Simplex Virus, ein Hepatitis-B Virus oder ein Hepatitis C-Virus ist.

8. Vektor nach den Ansprüchen 1 bis 7, **dadurch gekennzeichnet, dass** das Insert in die E1- und/oder die E3-Region eines rekombinanten Adenovirus einkloniert ist.

9. Vektor nach einem der Ansprüche 1 bis 8, **dadurch gekennzeichnet, daß** er durch homologe Rekombination eines viralen Plasmids und eines Expressionsplasmids mit der in SEQ ID NO:1, SEQ ID NO:2 oder SEQ ID NO:3 dargestellten Nukleinsäuresequenz erhältlich ist.

10. Expressionsplasmid mit der in SEQ ID NO:4 oder SEQ ID NO:5 dargestellten Nukleinsäuresequenz.

11. Verwendung eines Plasmids nach Anspruch 10 zur Herstellung eines Vektors nach den Ansprüchen 1 bis 9.

12. Verwendung der Vektoren nach den Ansprüchen 1 bis 9 zur *in vitro*-Genexpression in eukaryoten Zelllinien.

13. Verwendung der Vektoren nach den Ansprüchen 1 bis 9, bei denen 'Transgen' für ein therapeutisch wirksames Protein kodiert, zur Herstellung eines Medikamentes zur Gentherapie.

14. Verwendung nach Anspruch 13, bei dem das Transgen IL-2, IL-12, IL-18, TNF-α oder IFN-γ und die Gentherapie die Gentherapie maligner Erkrankungen ist.

15. Verwendung nach Anspruch 14, **dadurch gekennzeichnet, daß** die maligne Erkrankung ein solider Tumor ist.

16. Verwendung nach den Ansprüchen 12 bis 15, **dadurch gekennzeichnet, dass** man die Genexpression mit Doxycyclin, Tetracyclin, Oxytetracyclin, Chlortetracyclin, Demeclocyclin, Methacyclin oder Minocyclin, reguliert.

17. Verwendung der Vektoren nach den Ansprüchen 1 bis 9, bei denen 'Transgen' für ein Reporterprotein kodiert, zum Nachweis von Tetracyclin oder einem Derivat desselben in biologischen, lebensmittelchemischen oder ähnlichen Proben.

18. Verwendung nach Anspruch 17, **dadurch gekennzeichnet, dass** das Derivat Doxycyclin ist.

## Claims

1. Recombinant viral vector which contains an insert exhibiting the general structure
tTA - intron¹ - TK⁺ - TetO₇ - CMV⁺ - intron² - transgene
in which
TetO₇ is the heptamerized tetracycline operator
TK⁺ is the minimal thymidine kinase promoter
tTA is a nucleic acid sequence which encodes a fusion protein from the repressor protein inducible by tetracycline and the transcriptional activation domain of the Herpes simplex virus VP16,
CMV⁺ is the minimal cytomegalovirus promoter and
Transgene is a nucleic acid sequence which codes for a non-viral protein
Intron¹ is any desired non-encoding nucleic acid sequence with a length of O to approximately 1000 bp and
Intron² is any desired non-encoding nucleic acid sequence with a length of O to approximately 1000 bp.

2. Vector according to claim 1 **characterized in that** the insert is inserted into the viral vector genome in reverse orientation.

3. Vector according to claim 1 or 2 **characterized in that** the positions of tTA and transgene are inverted in the insert.

4. Vector according to claims 1 to 3 **characterized in that** the insert contains an additional lac repressor (lacR) between "CMV⁺" and "intron²" or between "intron²" and "transgene".

5. Vector according to claims 1 to 4 **characterized in that** the transgene is a nucleic acid sequence encoding a fluorescence protein, luciferase, interleukin-12 (IL-12), interleukin-18 (IL-18), interleukin-2 (IL-2), tumor necrosis factor α (TNF-α) or interferon-γ (IFN-γ).

6. Vector according to claim 5 **characterized in that** IL-12 is a single chain interleukin-12.

7. Vector according to claims 1 to 6 **characterized in that** the virus is an adenovirus, an adeno-associated adenovirus (AAV), a retrovirus, in particular a human immunodeficiency virus (HIV), a Herpes simplex virus, a Hepatitis B virus or Hepatitis C virus.

8. Vector according to claims 1 to 7 **characterized in that** the insert is cloned into the E1 and/or the E3 region of a recombinant adenovirus.

9. Vector according to claims 1 to 8 **characterized in that** it is obtainable by homologous recombination of a viral plasmid and an expression plasmid with the nucleic acid sequence represented in SEQ ID NO:1, SEQ ID NO:2 or SEQ ID NO:3.

10. Expression plasmid with the nucleic acid sequence represented in SEQ ID NO:4 or SEQ ID NO:5.

11. Use of a plasmid according to claim 10 for the production of a vector according to claims 1 to 9.

12. Use of the vectors according to claims 1 to 9 for the in vitro gene expression in eukaryotic cell lines.

13. Use of the vectors according to claim 1 to 9 wherein "transgene" encodes a therapeutically effective protein, for the preparation of a medicament for gene therapy.

14. Use according to claim 13 in which the transgene is IL-2, IL-12, IL-18, TNF-α or INF-γ, and the gene therapy is the gene therapy of malignant diseases.

15. Use according to claim 14 **characterized in that** the malignant disease is a solid tumor.

16. Use according to claims 12 to 15 **characterized in that** the gene expression is regulated with doxycycline, tetracycline, oxytetracycline, chlorotetracycline, demeclocycline, methacycline or minocycline.

17. Use of the vectors according to claims 1 to 9 wherein "transgene" encodes a reporter protein, for the detection of tetracycline or a derivative thereof in biological, food chemical or similar samples.

18. Use according to claim 17 **characterized in that** the derivative is doxycycline.

## Revendications

1. Vecteur viral recombinant qui contient un insert présentant la structure générale tTA - intron¹- TK⁺- TetO₇ - CMV⁺ - intron² - transgène
dans lequel
TetO₇ est un opérateur de tétracycline heptamérisé,
TK⁺ est le promoteur de thymidine kinase minimal,
tTA est une séquence d'acide nucléique qui code pour une protéine de fusion de la protéine répresseur inductible par la tétracycline et le domaine transcriptionnel du virus de l'herpès simplex VP16,
CMV⁺ est le promoteur du cytomégalovirus minimal et
transgène est une séquence d'acide nucléique codant pour une protéine non virale,
intron¹ est une séquence d'acide nucléique non codante quelconque, présentant une longueur de 0 à environ 1000 pb et
intron² est une séquence d'acide nucléique non codante quelconque présentant une longueur de 0 à environ 1000 pb.

2. Vecteur selon la revendication 1, **caractérisé en ce que** l'insert est inséré dans une orientation inverse dans le génome du vecteur viral.

3. Vecteur selon la revendication 1 ou 2, **caractérisé en ce que** les positions de tTA et du transgène dans l'insert sont échangées.

4. Vecteur selon les revendications 1 à 3, **caractérisé en ce que** l'insert entre "CMV⁺" et "Intron²" ou entre "Intron²" et "transgène" contient en outre un répresseur lac (lacR).

5. Vecteur selon les revendications 1 à 4, **caractérisé en ce que** le transgène est une séquence d'acide nucléique codant pour une protéine de fluorescence, pour la luciférase, pour l'interleukine 12 (IL-12), l'interleukine 18 (IL-18), l'interleukine 2 (IL-2), le facteur de nécrose tumoral α (TNF-α) ou l'interféron-γ (IFN-γ).

6. Vecteur selon la revendication 5, **caractérisé en ce que** IL-12 est une interleukine 12 à chaîne simple.

7. Vecteur selon les revendications 1 à 6, **caractérisé en ce que** le virus est un adénovirus, un adénovirus adéno-associé (AAV), un rétrovirus, en particulier un virus de l'immunodéficience humaine (VIH), un virus de l'herpès simplex, un virus de l'hépatite B ou un virus de l'hépatite C.

8. Vecteur selon les revendications 1 à 7, **caractérisé en ce que** l'insert est cloné dans la région E1 et/ou E3 d'un adénovirus recombinant.

9. Vecteur selon l'une des revendications 1 à 8, **caractérisé en ce qu'**il est obtenu par recombinaison homologue d'un plasmide viral et d'un plasmide d'expression avec la séquence d'acide nucléique présentée dans SEQ ID N° : 1, SEQ ID N° : 2 ou SEQ ID N° : 3.

10. Plasmide d'expression présentant la séquence d'acide nucléique présentée dans SEQ ID N° : 4 ou SEQ ID N° : 5.

11. Utilisation d'un plasmide selon la revendication 10, pour produire un vecteur selon les revendications 1 à 9.

12. Utilisation des vecteurs selon les revendications 1 à 9, pour l'expression génique in vitro dans des lignées de cellules eucaryotes.

13. Utilisation des vecteurs selon les revendications 1 à 9, dans lesquels "transgène" code pour une protéine thérapeutiquement efficace, pour la préparation d'un médicament pour la thérapie génique.

14. Utilisation selon la revendication 13, dans laquelle le transgène est IL-2, IL-12, IL-18, TNF-α ou IFN-γ et la thérapie génique est la thérapie génique d'affections malignes.

15. Utilisation selon la revendication 14, **caractérisée en ce que** l'affection maligne est une tumeur solide.

16. Utilisation selon les revendications 12 à 15, **caractérisée en ce que** l'expression génique est régulée avec la doxycycline, la tétracycline, l'oxytétracycline, la chlorotétracycline, la déméclocycline, la méthacycline ou la minocycline.

17. Utilisation des vecteurs selon les revendications 1 à 9, dans lesquels "transgène" code pour une protéine reporter, pour mettre en évidence la tétracycline ou un dérivé de celle-ci dans des échantillons biologiques, chimico-alimentaires ou autres échantillons comparables.

18. Utilisation selon la revendication 17, **caractérisée en ce que** le dérivé est la doxycycline.
